(19) Europäisches Patentamt
European Patent Office
Office européen des brevets

(11) EP 4 455 663 A1

(12) **EUROPEAN PATENT APPLICATION**
published in accordance with Art. 153(4) EPC

(43) Date of publication:
**30.10.2024 Bulletin 2024/44**

(21) Application number: **22915190.7**

(22) Date of filing: **30.12.2022**

(51) International Patent Classification (IPC):
***G01N 33/49*** *(2006.01)* ***G01N 15/00*** *(2024.01)*
***G01N 21/64*** *(2006.01)*

(52) Cooperative Patent Classification (CPC):
**G01N 15/00; G01N 21/64; G01N 33/49**

(86) International application number:
**PCT/CN2022/143971**

(87) International publication number:
**WO 2023/125942 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.12.2021 CN 202111674927**

(71) Applicant: **Shenzhen Mindray Bio-Medical Electronics Co., Ltd.**
**Shenzhen, Guangdong 518057 (CN)**

(72) Inventors:
- **LI, Jin**
  **Shenzhen, Guangdong 518057 (CN)**
- **ZHANG, Xiaomei**
  **Shenzhen, Guangdong 518057 (CN)**
- **PAN, Shiyao**
  **Shenzhen, Guangdong 518057 (CN)**
- **MA, Yushuai**
  **Shenzhen, Guangdong 518057 (CN)**
- **QI, Huan**
  **Shenzhen, Guangdong 518057 (CN)**
- **YE, Yi**
  **Shenzhen, Guangdong 518057 (CN)**

(74) Representative: **KIPA AB**
**Drottninggatan 11**
**252 21 Helsingborg (SE)**

# (54) BLOOD CELL ANALYZER, METHOD, AND USE OF INFECTION MARKER PARAMETER

(57) A blood cell analyzer, a method, and a use of an infection marker parameter. The blood cell analyzer includes: a sample aspiration device (110) for aspirating a blood sample of a subject to be tested, a sample preparation device (120) for preparing a test sample, an optical detection device (130) for detecting the test sample to obtain optical information, and a processor (140). The processor is configured to: obtain at least one leukocyte parameter of at least one leukocyte particle population in the test sample from the optical information, obtain an infection marker parameter on the basis of the at least one leukocyte parameter, and output the infection marker parameter, the infection marker parameter being used for predicting whether the subject is likely to progress to sepsis within a certain time period starting from when the blood sample to be tested is collected. Therefore, risk of sepsis can be quickly and accurately predicted in advance.

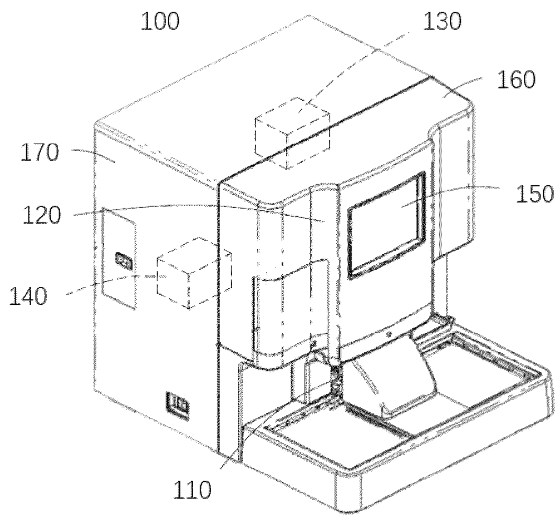


*FIG. 1*

EP 4 455 663 A1

## Description

### TECHNICAL FIELD

The disclosure relates to the field of in vitro diagnostics and, in particular, to a blood cell analyzer, a method for predicting whether a subject is likely to progress to sepsis in the future, and the use of an infection marker parameter in predicting whether a subject is likely to progress to sepsis in the future.

### BACKGROUND

Infectious diseases are common clinical diseases, among which sepsis is a serious infectious disease. The incidence of sepsis is high, with more than 18 million severe sepsis cases worldwide every year. Sepsis is dangerous and has a high case fatality rate, with about 14,000 people dying from its complications worldwide every day. According to foreign epidemiological surveys, the case fatality rate of sepsis has exceeded that of myocardial infarction, and has become the main cause of death for non-heart disease patients in intensive care units. In recent years, despite advances in anti-infective treatment and organ function support technologies, the case fatality rate of sepsis is still as high as 30% to 70%. The treatment of sepsis is expensive and consumes a lot of medical resources, which seriously affects the quality of human life and has posed a huge threat to human health.

To this end, clinicians need to diagnose whether the patient is infected in time and find the pathogen in order to make an effective treatment plan. Therefore, how to quickly and early screen and diagnose infectious diseases has become an urgent problem to be solved in clinical laboratories.

For rapid differential diagnosis of infectious diseases, existing solutions in the industry and their disadvantages are as follows:

1. Microbial culture: Microbial culture is considered to be the most reliable gold standard. It enables direct culture and detection of bacteria in clinical specimens such as body fluid or blood, so as to interpret the type and drug resistance of a bacteria, thereby providing direct guidance for clinical drug use. However, this microbial culture method has a long reporting cycle, specimens are easily contaminated and false negative rate is high, which cannot meet requirements of rapid and accurate clinical results.
2. Detection of inflammatory markers such as C-reactive protein (CRP), procalcitonin (PCT) and serum amyloid A (SAA): Inflammatory factors such as CRP, PCT and SAA are widely used in auxiliary diagnosis of infectious diseases due to their good sensitivity. However, respective specificity of these inflammatory markers is weak, and additional examination fees would occur, which increases financial burden on patients. In addition, CRP and PCT may be interfered by specific diseases and cannot correctly reflect infection status of patients. For example, CRP is generated in liver, and a level of CRP in infected patients with liver injury is normal, which may lead to false negatives.
3. Serum antigen and antibody detection: Serum antigen and antibody detection may identify specific virus types, but it has limited effect on situations where type of pathogen is not clear, and detection cost is high, necessitating additional fees for the examination, thereby increasing financial burden on patients.
4. Blood routine test: Blood routine test may indicate occurrence of infection and identify infection types to a certain extent. However, blood routine WBC\Neu% currently used in clinical practice is affected by many aspects, such as being easily affected by other non-infectious inflammatory responses, normal physiological fluctuations of body, etc., and cannot accurately and timely reflect patient's condition, and has poor diagnostic and therapeutic value in infectious diseases.

### SUMMARY

In order to at least partially solve the above-mentioned technical problems, an object of the disclosure is to provide a blood cell analyzer, a method for predicting whether a subject is likely to progress to sepsis in the future, and a use of an infection marker parameter in predicting whether a subject is likely to progress to sepsis in the future, which can obtain an infection marker parameter with high diagnostic efficacy from original signals during blood routine test process, so that it can accurately and quickly predict whether a subject is likely to develop to a septic patient in the future based on the infection marker parameter.

In order to achieve the above object of the disclosure, a first aspect of the disclosure provides a blood cell analyzer including:

a sample aspiration device configured to aspirate a blood sample of a subject to be tested;
a sample preparation device configured to prepare a test sample containing a part of the blood sample to be tested, a hemolytic agent, and a staining agent for leukocyte classification;

an optical detection device including a flow cell, a light source and an optical detector, the flow cell being configured to allow the test sample to pass therethrough, the light source being configured to irradiate with light the test sample passing through the flow cell, and the optical detector being configured to detect optical information generated by the test sample under irradiation when passing through the flow cell; and
a processor configured to:

calculate at least one leukocyte parameter of at least one leukocyte particle population in the test sample from the optical information,
obtain an infection marker parameter based on the at least one leukocyte parameter, and
output the infection marker parameter, which is used for predicting whether the subject is likely to progress to sepsis within a certain period of time starting from when the blood sample to be tested is collected.

In order to achieve the above object of the disclosure, a second aspect of the disclosure further provides a method for predicting whether a subject is likely to progress to sepsis in the future, including:

obtaining a blood sample to be tested from the subject;
preparing a test sample containing a part of the blood sample to be tested, a hemolytic agent, and a staining agent for leukocyte classification;
passing particles in the test sample through an optical detection region irradiated with light one by one, to obtain optical information generated by the particles in the test sample after being irradiated with light;
calculating at least one leukocyte parameter of at least one leukocyte particle population in the test sample from the optical information;
obtaining an infection marker parameter based on the at least one leukocyte parameter; and
predicting from the infection marker parameter whether the subject is likely to progress to sepsis within a certain period of time starting from when the blood sample to be tested is collected.

In order to achieve the above object of the disclosure, a third aspect of the disclosure further provides a use of the infection marker parameter in predicting whether the subject is likely to progress to sepsis in the future, wherein the infection marker parameter is obtained by:

obtaining at least one leukocyte parameter of at least one leukocyte particle population obtained by flow cytometry detection of a test sample containing a part of a blood sample to be tested from the subject, a hemolytic agent, and a staining agent for leukocyte classification; and
obtaining an infection marker parameter based on the at least one leukocyte parameter.

In the technical solutions provided in the various aspects of the disclosure, an infection marker parameter is calculated based on at least one leukocyte parameter obtained from a detection channel for leukocyte classification, and based on the infection marker parameter, it is possible to effectively predict whether the subject is likely to develop into a septic patient in the future, so that it is possible to quickly, accurately and efficiently assist a doctor in determining whether the subject is likely to develop into a septic patient.

BRIEF DESCRIPTION OF THE DRAWINGS

The disclosure will be more clearly explained below with reference to the embodiments and the accompanying drawings. The above and other advantages will become apparent to those of ordinary skill in the art from the detailed description of the embodiments of the disclosure. The accompanying drawings are only for illustrating preferred embodiments and should not be considered as limiting the disclosure. The same or similar reference numerals represent the same components throughout the accompanying drawings. In the accompanying drawings:

FIG. 1 is a schematic diagram of a structure of a blood cell analyzer according to some embodiments of the disclosure.
FIG. 2 is a schematic diagram of a structure of an optical detection device according to some embodiments of the disclosure.
FIG. 3 is an SS-FL two-dimensional scattergram of a test sample according to some embodiments of the disclosure.
FIG. 4 is an SS-FS two-dimensional scattergram of a test sample according to some embodiments of the disclosure.
FIG. 5 is an SS-FS-FL three-dimensional scattergram of a test sample according to some embodiments of the disclosure.
FIG. 6 shows cell characteristic parameters of neutrophil population in a test sample according to some embodiments of the disclosure.

FIG. 7 is a scattergram showing presence of abnormalities in a test sample according to some embodiments of the disclosure.

FIG. 8 is a schematic flowchart of a method for predicting whether a subject is likely to progress to sepsis in the future according to some embodiments of the disclosure.

FIGS. 9-10 are ROC curves of infection marker parameters when used to predict whether a subject is likely to progress to sepsis in the future, according to some embodiments of the disclosure.

FIG. 11 shows calculation steps of an algorithm of an area parameter D_NEU_FLSS_Area of neutrophil population according to some embodiments of the disclosure.

DESCRIPTION OF THE PREFERRED EMBODIMENTS

The embodiments of the disclosure will be described below clearly and completely with reference to the accompanying drawings. Obviously, the embodiments described are merely some, rather than all, of the embodiments of the disclosure. Based on the embodiments of the disclosure, all the other embodiments which would have been obtained by those of ordinary skill in the art without any creative efforts shall fall within the scope of protection of the disclosure.

It should be noted that the term "first/second/third" in the embodiments of the disclosure is only used to distinguish similar objects, and does not represent specific order for the objects. It may be understood that "first/second/third" may be interchanged for specific order or chronological order when allowed.

It can be understood by those skilled in the art that unless otherwise defined, all terms (including technical terms and scientific terms) used herein have the same meanings as those generally understood by those of ordinary skill in the art to which the disclosure belongs.

1) Scattergram: it is a two-dimensional or three-dimensional diagram generated by a blood cell analyzer, with two-dimensional or three-dimensional feature information about a plurality of particles distributed thereon, wherein X coordinate axis, Y coordinate axis and Z coordinate axis of the scatter diagram each represent a characteristic of each particle. For example, in a scatter diagram, X coordinate axis represents forward scatter intensity, Y coordinate axis represents fluorescence intensity, and Z coordinate axis represents side scatter intensity. The term "scattergram" used in the present disclosure refers not only to a distribution map of at least two sets of data in a rectangular coordinate system in the form of data points, but also to an array of data, that is, not limited by its graphical presentation form.

2) particle population/cell population: it is distributed in a certain region of a scattergram, and is a particle cluster formed by a plurality of particles having identical cell characteristics, such as leukocyte (including all types of leukocytes) population, and leukocyte subpopulation, such as neutrophil population, lymphocyte population, monocyte population, eosinophil population, or basophil population.

3) Blood ghost: they are fragmented particles obtained by dissolving red blood cells and blood platelets in blood with a hemolytic agent.

4) ROC curve (receiver operating characteristic curve): it is receiver operating characteristic curve, which is a curve plotted based on a series of different binary classifications (discrimination thresholds), with true positive rate as ordinate and false positive rate as abscissa, and ROC_AUC represents an area enclosed by ROC curve and horizontal coordinate axis.

ROC curve is plotted by setting a number of different critical values for continuous variables, calculating a corresponding sensitivity and specificity at each critical value, and then plotting a curve with sensitivity as vertical coordinate and 1-specificity as horizontal coordinate.

Because ROC curve is composed of multiple critical values representing their respective sensitivity and specificity, a best diagnostic threshold value for a certain diagnostic method can be selected with the help of ROC curve. The closer the ROC curve is to the upper left corner, the higher the test sensitivity and the lower the misjudgment rate, the better the performance of the diagnosis method. It can be seen that the point on the ROC curve closest to the upper left corner of the ROC curve has the largest sum of sensitivity and specificity, and the value corresponding to this point or its adjacent points is often used as a diagnostic reference value (also known as a diagnostic threshold)

Currently, a blood cell analyzer generally counts and classifies leukocytes through a DIFF channel and/or a WNB channel. The blood cell analyzer performs a four-part differential of leukocytes via the DIFF channel, and classifies leukocytes into four types of leukocytes: lymphocytes (Lym), monocytes (Mon), neutrophils (Neu), and eosinophils (Eos). The blood cell analyzer identifies nucleated red blood cells through the WNB channel, and can obtain a nucleated red blood cell count, a leukocyte count, and a basophil count at the same time. A combination of the DIFF channel and the WNB channel results in a five-part differential of leukocytes, including five types of leukocytes: lymphocytes (Lym), monocytes (Mon), neutrophils (Neu), eosinophils (Eos), and basophils (Baso).

The blood cell analyzer used in the disclosure implements classification and counting of particles in a blood sample through a flow cytometry technique combined with a laser scattering method and a fluorescence staining method. Here, the principle of testing a blood sample by the blood cell analyzer may be, for example: first, a blood sample is aspirated and treated with a hemolytic agent and a fluorescent dye, wherein red blood cells are destroyed and dissolved by the hemolytic agent, while white blood cells will not be dissolved, but the fluorescent dye can enter white blood cell nucleus with the help of the hemolytic agent and then is bound with nucleic acid substance of the nucleus; and then, particles in the sample are made to pass through a detection aperture irradiated by a laser beam one by one. When the laser beam irradiates the particles, properties (such as volume, degree of staining, size and content of cell contents, density of cell nucleus) of the particles themselves may block or change a direction of the laser beam, thereby generating scattered light at various angles that corresponds to their properties, and the scattered light can be received by a signal detector to obtain relevant information about structure and composition of the particles. Forward-scattered light (FS) reflects a number and a volume of particles, side-scattered light (SS) reflects a complexity of a cell internal structure (such as intracellular particle or nucleus), and fluorescence (FL) reflects a content of nucleic acid substance in a cell. The use of the light information can implement differential and counting of the particles in the sample.

FIG. 1 is a schematic diagram of a structure of a blood cell analyzer according to some embodiments of the disclosure. The blood cell analyzer 100 includes a sample aspiration device 110, a sample preparation device 120, an optical detection device 130, and a processor 140. The blood cell analyzer 100 further has a liquid circuit system (not shown) for connecting the sample aspiration device 110, the sample preparation device 120, and the optical detection device 130 for liquid transport between these devices.

The sample aspiration device 110 is configured to aspirate a blood sample of a subject to be tested.

In some embodiments, the sample aspiration device 110 has a sampling needle (not shown) for aspirating a blood sample to be tested. In addition, the sample aspiration device 110 may further include, for example, a driving device configured to drive the sampling needle to quantitatively aspirate the blood sample to be tested through a needle nozzle of the sampling needle. The sample aspiration device 110 can transport the aspirated blood sample to the sample preparation device 120.

The sample preparation device 120 is at least configured to prepare a test sample containing a part of the blood sample to be tested, a hemolytic agent, and a staining agent for leukocyte classification.

In embodiments of the disclosure, the hemolytic agent herein is configured to lyse red blood cells in blood to break the red blood cells into fragments, with morphology of leukocytes substantially unchanged.

In some embodiments, the hemolytic agent may be any one or a combination of a cationic surfactant, a non-ionic surfactant, an anionic surfactant and an amphiphilic surfactant. In other embodiments, the hemolysis reagent may include at least one of alkyl glycosides, triterpenoid saponins and steroidal saponins.

In embodiments of the disclosure, the staining agent is a fluorescent dye used for achieving leukocyte differential count, for example, is a fluorescent dye that can achieve differential count of the leukocytes in a blood sample into at least three leukocyte subpopulations (monocytes, lymphocytes and neutrophils).

In some embodiments, the staining agent may include a membrane-specific dye or a mitochondrial-specific dye. For more details, reference may be made to the PCT patent application WO 2019/206300 A1 filed by the applicant on April 26, 2019, which is incorporated herein by reference in its entirety.

In other embodiments, the staining agent may comprise a cationic cyanine compound. For more details, reference may be made to Chinese Patent Application CN 101750274 A filed by the Applicant on September 28, 2019, the entire disclosure of which is incorporated herein by reference.

In some embodiments, the sample preparation device 120 may comprise at least one reaction cell and a reagent supply device (not shown). The at least one reaction cell is configured to receive the blood sample to be tested aspirated by the sample aspiration device 110, and the reagent supply device supplies treatment reagents (including the hemolytic reagent, the staining agents, etc.) to the at least one reaction cell, so that the blood sample to be tested aspirated by the sample aspiration device 110 is mixed, in the reaction cell, with the treatment reagents supplied by the reagent supply device to prepare a test sample.

For example, the at least one reaction cell may include a first reaction cell and a second reaction cell, and the reagent supply device may include a first reagent supply portion and a second reagent supply portion. The sample aspiration device 110 is configured to respectively dispense the aspirated blood sample to be tested in part respectively to the first reaction cell and the second reaction cell. The first reagent supply portion is configured to supply a first hemolytic agent and a first staining agent for leukocyte classification to the first reaction cell, so that part of the blood sample to be tested that is dispensed to the first reaction cell is mixed and reacts with the first hemolytic agent and the first staining agent so as to prepare a first test sample. The second reagent supply portion is configured to supply a second hemolytic agent and a second staining agent for identifying nucleated red blood cells to the second reaction cell, so that part of the test blood sample that is dispensed to the second reaction cell is mixed and reacts with the second hemolytic agent and the second staining agent so as to prepare a second test sample.

The optical detection device 130 includes a flow cell, a light source and an optical detector, wherein the flow cell is configured to allow the test sample to pass therethrough, the light source is configured to irradiate with light the test sample passing through the flow cell, and the optical detector is configured to detect optical information generated by the test sample under irradiation when passing through the flow cell.

For example, the first test sample and the second test sample pass through the flow cell respectively, and the light source irradiates the first test sample and the second test sample passing through the flow cell respectively. The optical detector is used for detecting first optical information and second optical information generated after the first test sample and the second test sample are irradiated with light when they pass through the flow cell, respectively.

It will be understood herein that the first detection channel for leukocyte classification (also referred to as DIFF channel) refers to the detection by the optical detection device 130 of the first test sample prepared by the sample preparation device 120, and the second detection channel for identifying nucleated red blood cells (also referred to as WNB channel) refers to the detection by the optical detection device 130 of the second test sample prepared by the sample preparation device 120.

Herein, the flow cell refers to a cell that focuses flow and is suitable for detecting light scattering signals and fluorescence signals. When a particle, such as a blood cell, passes through a detection aperture of the flow cell, the particle scatters, to various directions, an incident light beam from the light source directed to the detection aperture. An optical detector may be provided at one or more different angles relative to the incident light beam, to detect light scattered by the particle to obtain scattered light signals. Since different particles have different light scattering properties, the light scattering signals can be used to distinguish between different particle clusters. Specifically, light scattering signals detected in the vicinity of the incident beam are often referred to as forward light scattering signals or small-angle light scattering signals. In some embodiments, forward light scattering signals can be detected at an angle of about 1° to about 10° from the incident beam. In some other embodiments, forward light scattering signals can be detected at an angle of about 2° to about 6° from the incident beam. Light scattering signals detected at about 90° from the incident beam are commonly referred to as side light scattering signals. In some embodiments, side light scattering signals can be detected at an angle of about 65° to about 115° from the incident beam. Typically, fluorescence signals from a blood cell stained with a fluorescent dye are also generally detected at about 90° from the incident beam.

In some embodiments, the optical detector may include a forward scatter detector for detecting forward scatter signals (or forward scatter intensity), a side scatter detector for detecting side scatter signals (or side scatter intensity), and a fluorescence detector for detecting fluorescence signals (or fluorescence intensity). Accordingly, the optical information may include forward scatter signals, side scatter signals, and fluorescence signals of particles in the test sample.

FIG. 2 shows a specific example of the optical detection device 130. The optical test apparatus 130 is provided with a light source 101, a beam shaping assembly 102, a flow cell 103 and a forward-scattered light detector 104 which are sequentially arranged in a straight line. On one side of the flow cell 103, a dichroscope 106 is arranged at an angle of 45° to the straight line. Part of lateral light emitted by particles in the flow cell 103 is transmitted through the dichroscope 106 and is captured by a fluorescence detector 105 arranged behind the dichroscope 106 at an angle of 45° to the dichroscope 106; and the other part of the lateral light is reflected by the dichroscope 106 and is captured by a side-scattered light detector 107 arranged in front of the dichroscope 106 at an angle of 45° to the dichroscope 106.

The processor 140 is configured to process and operate data to obtain a required result. For example, the processor may be configured to generate a two-dimensional scattergram or a three-dimensional scattergram based on various collected light signals, and perform particle analysis using a method of gating on the scattergram. The processor 140 may also be configured to perform visualization processing on an intermediate operation result or a final operation result, and then display same by a display apparatus 150. In embodiments of the disclosure, the processor 140 is configured to implement methods and steps which will be described in detail below.

In embodiments of the present disclosure, the processor includes, but is not limited to, a central processing unit (CPU), a micro controller unit (MCU), a field-programmable gate array (FPGA), a digital signal processor (DSP) and other devices for interpreting computer instructions and processing data in computer software. For example, the processor is configured to execute each computer application program in a computer-readable storage medium, so that the blood cell analyzer 100 preforms a corresponding detection process and analyzes, in real time, optical information or optical signals detected by the optical detection device 130.

In addition, the blood cell analyzer 100 may further include a first housing 160 and a second housing 170. The display apparatus 150 may be, for example, a user interface. The optical detection apparatus 130 and the processor 140 are provided inside the second housing 170. The sample preparation apparatus 120 is provided, for example, inside the first housing 160, and the display apparatus 150 is provided, for example, on an outer surface of the first housing 160 and configured to display test results from the blood cell analyzer.

As mentioned in the BACKGROUND, blood routine tests realized by using the blood cell analyzer can indicate occurrence of infection and identify infection types, but blood routine WBC/Neu% currently used in clinical practice is affected by many aspects and cannot accurately and timely reflect patient condition. Moreover, sensitivity and specificity of the existing technology in diagnosis and treatment of bacterial infection and sepsis are poor.

Sepsis is a serious infectious disease with high incidence and case fatality rate. Every hour of delay in treatment, the mortality rate of patients increases by 7%. Therefore, early warning of sepsis is particularly important. Early identification and early warning of sepsis can increase the precious diagnosis and treatment time for patients and greatly improve the survival rate.

On this base, by intensively studying original signal characteristics of blood routine tests of a large number of blood samples from sepsis patients, the inventors found that high- efficacy early prediction of sepsis can be realized by at least one leukocyte parameter, especially leukocyte characteristic parameter, obtained from DIFF channel. For example, neutrophils and monocytes are the body's first barrier against infection and are valuable in reflecting infection degree. Through research, the inventors found that a characteristic parameter of neutrophils can be used for early prediction of sepsis, and further, a characteristic parameter of neutrophils combined with a characteristic parameter of monocytes can achieve higher efficacy in early prediction of sepsis.

A leukocyte parameter of the DIFF channel, especially a leukocyte characteristic parameter, may realize high efficacy in early prediction of sepsis through, for example, linear discriminant analysis (LDA). The linear discriminant analysis is an induction of Fisher's linear discriminant method, which uses statistics, pattern recognition, and machine learning methods to characterize or distinguish two types of events (e.g., whether a donor will or will not progress to sepsis within a certain period of time in the future, a donor is suffering from sepsis or is not suffering from sepsis, a donor is suffering from bacterial infection or viral infection, a donor is suffering from infectious inflammation or is suffering from non-infectious inflammation, and whether sepsis treatment is effective or ineffective) by finding a linear combination of characteristics of the two types of events and by obtaining one-dimensional data via linearly combining multi-dimensional data. The coefficient of the linear combination can ensure that the degree of discrimination of the two types of events is maximized. The resulting linear combination can be used to classify subsequent events.

Therefore, an embodiment of the disclosure first proposes a blood cell analyzer 100, including:

a sample aspiration device 110 configured to aspirate a blood sample of a subject to be tested;

a sample preparation device 120 for preparing a test sample containing a part of the blood sample to be tested, a hemolytic agent, and a staining agent for leukocyte classification;

an optical detection device 130 including a flow cell, a light source and an optical detector, the flow cell being configured to allow the test sample to pass therethrough, the light source being configured to irradiate with light the test sample passing through the flow cell, and the optical detector being configured to detect optical information generated by the test sample under irradiation when passing through the flow cell; and

a processor 140 configured to:

calculate at least one leukocyte parameter of at least one leukocyte particle population in the test sample from the optical information,

obtain an infection marker parameter based on the at least one leukocyte parameter, and

output the infection marker parameter for predicting whether the subject is likely to progress to sepsis within a certain period of time starting from when the blood sample to be tested is collected.

Herein, the certain period of time is, for example, not greater than 48 hours, preferably not greater than 24 hours. For example, the certain period of time may be between 24 hours and 48 hours.

Preferably, the at least one leukocyte parameter includes a cell characteristic parameter, i.e., the at least one leukocyte parameter includes a cell characteristic parameter of the at least one leukocyte particle population. Thus, an infection marker parameter with further improved diagnostic efficacy can be provided.

It should be understood that a cell characteristic parameter of the leukocyte particle population does not include a cell count or classification parameter of the leukocyte particle population, but include a characteristic parameter reflecting cell characteristics such as volume, internal granularity, internal nucleic acid content of cells in the leukocyte particle population.

In some embodiments, a cell characteristic parameter of the leukocyte particle population may be obtained by analyzing information of all the particles of the leukocyte particle population, or may be obtained by analyzing information of a part of particles of the leukocyte particle population. For example, a cell characteristic parameter of the leukocyte particle population can be obtained by identifying particle information that may carry infection characteristic information in the non-overlapping part of the leukocyte particle populations between the sample to be tested and blood samples of healthy individuals.

Further, in some embodiments, leukocytes in the test sample may be classified, based on the optical information, at least into monocyte population, neutrophil population and lymphocyte population, and in particular into monocyte population, neutrophil population, lymphocyte population and eosinophil population.

In one specific example, as shown in FIGS. 3 to 5, the leukocytes in the test sample may be classified into monocyte population Mon, neutrophil population Neu, lymphocyte population Lym, and eosinophil population Eos based on forward scatter signals (or the forward scatter intensity) FS, side scatter signals (or the side scatter intensity) SS, and fluorescence signals (or the fluorescence intensity) FL in the optical information. FIG. 3 is a two-dimensional scattergram generated based on the side scatter signals SS and the fluorescent signals FL in the optical information, FIG. 4 is a two-dimensional scattergram generated based on the forward scatter signals FS and the side scatter signals SS in the optical information, and FIG. 5 is a three-dimensional scattergram generated based on the forward scatter signals FS, the side scatter signals SS and the fluorescent signals FL in the optical information.

Accordingly, in some embodiments, the at least one leukocyte particle population may include at least one cell population of monocyte population Mon, neutrophil population Neu, lymphocyte population Lym and eosinophil population Eos in the test sample, i.e., the at least one leukocyte parameter may include one or more parameters of the cell characteristic parameters of monocyte population Mon, neutrophil population Neu, lymphocyte population Lym and eosinophil population Eos in the test sample.

Preferably, the at least one leukocyte parameter includes one or more of cell characteristic parameters of monocyte population Mon, neutrophil population Neu and lymphocyte population Lym in the test sample.

More preferably, the at least one leukocyte particle population may include at least one cell population of monocyte population Mon and neutrophil population Neu in the test sample, i.e., the at least one leukocyte parameter may include one or more parameters, e.g., one or two or more parameters, of cell characteristic parameters of monocyte population Mon and neutrophil population Neu in the test sample.

In some embodiments, the at least one leukocyte parameter includes one or more of following parameters: A forward scatter intensity distribution width, a forward scatter intensity distribution center of gravity, a forward scatter intensity distribution variation coefficient, a side scatter intensity distribution width, a side scatter intensity distribution center of gravity, a side scatter intensity distribution coefficient of variation, a fluorescence intensity distribution width, a fluorescence intensity distribution center of gravity, a fluorescence intensity distribution coefficient of variation of the leukocyte particle population, and an area of a distribution region of the leukocyte particle population in a two-dimensional scattergram generated by two light intensities selected from forward scatter intensity, side scatter intensity and fluorescence intensity, and a volume of a distribution region of the leukocyte particle population in a three-dimensional scattergram generated by forward scatter intensity, side scatter intensity and fluorescence intensity. In some specific examples, the at least one leukocyte parameter may include one or more, e.g. one or two parameters, of following parameters: a forward scatter intensity distribution width D_MON_FS_W, a forward scatter intensity distribution center of gravity D_MON_FS_P, a forward scatter intensity distribution coefficient of variation D_MON_FS_CV, a side scatter intensity distribution width D_MON_SS_W, a side scatter intensity distribution center of gravity D_MON_SS_P, a side scatter intensity distribution coefficient of variation D_MON_SS_CV, a fluorescence intensity distribution width D_MON_FL_W, a fluorescence intensity distribution center of gravity D_MON_FL_P, and a fluorescence intensity distribution coefficient of variation D_MON_FL_CV of monocyte population in the test sample, and an area D_MON_FLFS_Area (an area of a distribution region of monocyte population in a two-dimensional scattergram generated by forward scatter intensity and fluorescence intensity), D_MON_FLSS_Area (an area of a distribution region of monocyte population in a two-dimensional scattergram generated by side scatter intensity and fluorescence intensity), and D_MON_SSFS_Area (an area of a distribution region of monocyte population in a two-dimensional scattergram generated forward scatter intensity and side scatter intensity) of a distribution region of monocyte population in a two-dimensional scattergram generated by two light intensities selected from forward scatter intensity, side scatter intensity and fluorescence intensity, and a volume of a distribution region of monocyte population in a three-dimensional scattergram generated by forward scatter intensity, side scatter intensity and fluorescence intensity; a forward scatter intensity distribution width D_NEU_FS_W, a forward scatter intensity distribution center of gravity D_NEU_FS_P, a forward scatter intensity distribution coefficient of variation D_NEU_FS_CV, a side scatter intensity distribution width D_NEU_SS_W, a side scatter intensity distribution center of gravity D_NEU_SS_P, a side scatter intensity distribution coefficient of variation D_NEU_SS_CV, a fluorescence intensity distribution width D_NEU_FL_W, a fluorescence intensity distribution center of gravity D_NEU_FL_P, and a fluorescence intensity distribution coefficient of variation D_NEU_FL_CV of neutrophil population in the test sample, and an area D_NEU_FLFS_Area (an area of a distribution region of neutrophil population in a two-dimensional scattergram generated by forward scatter intensity and fluorescence intensity), D_NEU_FLSS_Area (an area of a distribution region of neutrophil population in a two-dimensional scattergram generated by side scatter intensity and fluorescence intensity), and D_NEU_SSFS_Area (an area of a distribution region of neutrophil population in a two-dimensional scattergram generated forward scatter intensity and side scatter intensity) of a distribution region of neutrophil population in a two-dimensional scattergram generated by two light intensities selected from forward scatter intensity, side scatter intensity and fluorescence intensity, and a volume of a distribution region of neutrophil population in a three-dimensional scattergram generated by forward scatter intensity, side scatter intensity and fluorescence intensity; and a forward scatter intensity distribution width D_LYM_FS_W, a forward scatter intensity distribution center of gravity D_LYM_FS_P, a forward scatter intensity distribution coefficient of variation D_LYM_FS_CV, a side scatter intensity distribution width D_LYM_SS_W, a side scatter intensity distribution center of gravity D_LYM_SS_P, a side scatter intensity distribution coefficient of variation D_LYM_SS_CV, a fluorescence intensity distribution width D_LYM_FL_W, a fluorescence intensity distribution center of gravity D_LYM_FL_P, and a fluorescence intensity distribution coefficient of variation D_LYM_FL_CV of lymphocyte population in the test sample, and an area D_LYM_FLFS_Area (an area of a distribution region of lymphocyte population in a two-dimensional scattergram generated by forward scatter intensity and fluorescence intensity), D_LYM_FLSS_Area (an area of a distribution region of lymphocyte population in a two-dimensional scattergram generated by side scatter intensity and fluorescence intensity), and D_LYM_SSFS_Area (an area of a distribution region of lymphocyte population in a two-dimensional scattergram generated forward scatter intensity and side scatter intensity) of a distribution region of lymphocyte population in a two-dimensional scattergram generated by two light intensities selected from forward scatter intensity, side scatter intensity and fluorescence intensity, and a volume of a distribution region of lymphocyte population in a three-dimensional scattergram generated by forward scatter intensity, side scatter intensity and fluorescence intensity; a forward scatter intensity distribution width D_EOS_FS_W, a forward scatter intensity distribution center of gravity D_EOS_FS_P, a forward scatter intensity distribution coefficient of variation D_EOS_FS_CV, a side scatter intensity distribution width D_EOS_SS_W, a side scatter intensity distribution center of gravity D_EOS_SS_P, a side scatter intensity distribution coefficient of variation D_EOS_SS_CV, a fluorescence intensity distribution width D_EOS_FL_W, a fluorescence intensity distribution center of gravity D_EOS_FL_P, and a fluorescence intensity distribution coefficient of variation D_EOS_FL_CV of eosinophil population in the test sample, and an area D_EOS_FLFS_Area (an area of a distribution region of eosinophil population in a two-dimensional scattergram generated by forward scatter intensity and fluorescence intensity), D_EOS_FLSS_Area (an area of a distribution region of eosinophil population in a two-dimensional scattergram generated by side scatter intensity and fluorescence intensity), and D_EOS_SSFS_Area (an area of a distribution region of eosinophil population in a two-dimensional scattergram generated forward scatter intensity and side scatter intensity) of a distribution region of eosinophil population in a two-dimensional scattergram generated by two light intensities selected from forward scatter intensity, side scatter intensity and fluorescence intensity, and an area of a distribution region of eosinophil population in a three-dimensional scattergram generated by forward scatter intensity, side scatter intensity and fluorescence intensity; and a forward scatter intensity distribution width D_WBC_FS_W, a forward scatter intensity distribution center of gravity D_WBC_FS_P, a forward scatter intensity distribution coefficient of variation D_WBC_FS_CV, a side scatter intensity distribution width D_WBC_SS_W, a side scatter intensity distribution center of gravity D_WBC_SS_P, a side scatter intensity distribution coefficient of variation D_WBC_SS_CV, a fluorescence intensity distribution width D_WBC_FL_W, a fluorescence intensity distribution center of gravity D_WBC_FL_P, and a fluorescence intensity distribution coefficient of variation D_WBC_FL_CV of leukocyte population in the test sample, and an area D_WBC_FLFS_Area (an area of a distribution region of leukocyte population in a two-dimensional scattergram generated by forward scatter intensity and fluorescence intensity), D_WBC_FLSS_Area (an area of a distribution region of leukocyte population in a two-dimensional scattergram generated by side scatter intensity and fluorescence intensity), and D_WBC_SSFS_Area (an area of a distribution region of leukocyte population in a two-dimensional scattergram generated forward scatter intensity and side scatter intensity) of a distribution region of leukocyte population in a two-dimensional scattergram generated by two light intensities selected from forward scatter intensity, side scatter intensity and fluorescence intensity, and a volume of a distribution region of leukocyte population in a three-dimensional scattergram generated by forward scatter intensity, side scatter intensity and fluorescence intensity.

Preferably, the at least one leukocyte parameter may include one or more, e.g. one or two parameters, of following parameters: a forward scatter intensity distribution width D_MON_FS_W, a forward scatter intensity distribution center of gravity D_MON_FS_P, a forward scatter intensity distribution coefficient of variation D_MON_FS_CV, a side scatter intensity distribution width D_MON_SS_W, a side scatter intensity distribution center of gravity D_MON_SS_P, a side scatter intensity distribution coefficient of variation D_MON_SS_CV, a fluorescence intensity distribution width D_MON_FL_W, a fluorescence intensity distribution center of gravity D_MON_FL_P, and a fluorescence intensity distribution coefficient of variation D_MON_FL_CV of monocyte population in the test sample, and areas D_MON_FLFS_Area, D_MON_FLSS_Area and D_MON_SSFS_Area of a distribution region of monocyte population in a two-dimensional scattergram generated by two light intensities selected form forward scatter intensity, side scatter intensity and fluorescence intensity, and a volume of a distribution region of monocyte population in a three-dimensional scattergram generated by forward scatter intensity, side scatter intensity and fluorescence intensity; and a forward scatter intensity distribution width D_NEU_FS_W, a forward scatter intensity distribution center of gravity D_NEU_FS_P, a forward scatter intensity distribution coefficient of variation D_NEU_FS_CV, a side scatter intensity distribution width D_NEU_SS_W, a side scatter intensity distribution center of gravity D_NEU_SS_P, a side scatter intensity distribution coefficient of variation D_NEU_SS_CV, a fluorescence intensity distribution width D_NEU_FL_W, a fluorescence intensity distribution center of gravity D_NEU_FL_P, and a fluorescence intensity distribution coefficient of variation D_NEU_FL_CV of neutrophil population in the test sample, and areas D_NEU_FLFS_Area, D_NEU_FLSS_Area, and D_NEU_SSFS_Area of a distribution region of neutrophil population in a two-dimensional scattergram generated by two light intensities selected from forward scatter intensity, side scatter intensity and fluorescence intensity, and a volume of a distribution region of neutrophil population in a three-dimensional scattergram generated by forward scatter intensity, side scatter intensity and fluorescence intensity.

In other embodiments, the at least one leukocyte parameter may also include a classification parameter Mon% or a count parameter Mon # of the monocyte population Mon or a classification parameter Neu% or a count parameter Neu # of the neutrophil population Neu or a classification parameter Lym% or a count parameter Mon # of the lymphocyte population Lym in the test sample.

The meanings of the distribution width, the distribution center of gravity, the coefficient of variation, and the area or volume of the distribution region are explained herein with reference to FIG. 6, wherein FIG. 6 shows cell characteristic parameters of neutrophil populations in the test sample according to some embodiments of the disclosure.

As shown in FIG. 6, D_NEU_FL_W represents the fluorescence intensity distribution width of the neutrophil population in the test sample, wherein D_NEU_FL_W is equal to the difference between the fluorescence intensity distribution upper limit S1 of the neutrophil population and the fluorescence intensity distribution lower limit S2 of the neutrophil population. D_NEU_FL_P represents the center of gravity of the fluorescence intensity distribution of the neutrophil population in the test sample, that is, the average position of the neutrophil population in the FL direction, wherein D_NEU_FL_P is calculated by the following formula:

$$\mathrm{D_NEU_FL_P} = \frac{\sum_1^N FL(i)}{N}$$

where FL (i) is the fluorescence intensity of the i-th neutrophil. D_NEU_FL_CV represents the coefficient of variation of the fluorescence intensity distribution of the neutrophil population in the test sample, where D_NEU_FL_CV is equal to D_NEU_FL_W divided by D_NEU_FL_P.

In addition, D_NEU_FLSS_Area represents the area of the distribution region of the neutrophil population in the test sample in the scattergram generated by side scatter intensity and fluorescence intensity.

In some embodiments, as shown in FIG. 6, C1 represents a contour distribution curve of the neutrophil population, for example, the total number of positions within the contour distribution curve C1 may be denoted as the area D_NEU_FLSS_Area of the neutrophil population.

In other embodiments, the D_NEU_FLSS_Area may also be implemented by the following algorithmic steps (FIG. 11):

randomly selecting a particle P1 from the neutrophil (NEU) particle population, and finding a particle P2 that is farthest from P1;
constructing a vector V1 (P1-P2), and taking P1 as the starting point of the vector, finding another particle P3 in the neutrophil (NEU) particle population, and constructing a vector V2 (P1-P3) such that the vector V2 (P1-P3) has a maximum angle with the vector V1 (P1-P2);
then, taking P1 as the starting point of the vector, finding another particle P4 in the neutrophil (NEU) particle population, and constructing a vector V3 (P1-P4) such that the vector V3 (P1-P4) has a maximum angle with the vector V1 (P1-P2);
by analogy, obtaining a group of particles P1, P2, P3, P4, ... Pn on the outermost side of the neutrophil (NEU) particle population, respectively;
fitting the particle points P1, P2, P3, P4, ... Pn by using an ellipse, and obtaining the major axis a and minor axis b of this ellipse;
the D_NEU_FLSS_Area is a product of the major axis a and the minor axis b.

Similarly, the volume parameter of the distribution region of the neutrophil population in the three-dimensional scattergram generated by forward scatter intensity, side scatter intensity and fluorescence intensity may also be obtained by corresponding calculations.

As will be appreciated herein, for definitions of other leukocyte parameters, reference may be made in a corresponding manner to the embodiments shown in FIGS. 6 and 11.

In some embodiments, the infection marker parameter may include a single leukocyte parameter, for example one of the cell characteristic parameters enumerated above. Alternatively, the infection marker parameter may be a linear function or a nonlinear function of a single leukocyte parameter.

In other embodiments, the infection marker parameter may also be calculated from a combination of at least two leukocyte parameters, i.e., the infection marker parameter is a function, e.g., a linear function, of at least two leukocyte parameters. At cell type level, for example, both neutrophils and monocytes are the first barrier of the body against infection, and both are valuable in reflecting infection degree. Therefore, a combination of a characteristic parameter of neutrophils and a characteristic parameter of monocytes can improve the predictive, diagnostic and/or guiding therapeutic efficacy of the present invention.

In other embodiments, the infection marker parameter may be calculated from the leukocyte parameter and another blood cell parameter, i.e., the infection marker parameter may be calculated from at least one leukocyte parameter and at least one other blood cell parameter. The other blood cell parameter may be a classification or counting parameter of platelets (PLTs), nucleated red blood cells (NRBCs), or reticulocytes (RETs).

To this end, in some embodiments, the processor 140 may be further configured to:

calculate at least one first leukocyte parameter of a first leukocyte particle population in the test sample and at least one second leukocyte parameter of a second leukocyte particle population in the test sample from the optical information; and

calculate the infection marker parameter based on the at least one first leukocyte parameter and the at least one second leukocyte parameter.

Herein, the first leukocyte particle population and the second leukocyte particle population are different from each other, and may be selected from the group consisting of monocyte population Mon, neutrophil population Neu, lymphocyte population Lym, and eosinophil population Eos in the test sample.

Preferably, the first leukocyte particle population is monocyte population and the second leukocyte particle population is neutrophil population. Accordingly, the at least one first leukocyte parameter preferably includes at least one cellular characteristic parameter of the monocyte population, and the at least one second leukocyte parameter preferably includes at least one cellular characteristic parameter of the neutrophil population.

Further preferably, the processor 140 may be further configured to combine the at least one first leukocyte parameter and the at least one second leukocyte parameter as the infection marker parameter by using a linear function, i.e., to calculate the infection marker parameter bt using the following formula:

$$Y=A*X1+B*X2+C$$

where Y represents the infection marker parameter, X1 represents the first leukocyte parameter, X2 represents the second leukocyte parameter, and A, B, and C are constants.

Of course, in other embodiments, the at least one first leukocyte parameter and the at least one second leukocyte parameter may also be combined as the infection marker parameter by using a nonlinear function, which is not specifically limited in the disclosure.

In other embodiments, the processor 140 may also be further configured to:

calculate at least two leukocyte parameters of one leukocyte particle population in the test sample from the optical information; and

calculate the infection marker parameter based on the at least two leukocyte parameters, in particular by using a linear function.

In some examples, infection marker parameters for predicting sepsis can be calculated, for example, with parameter combinations shown in Table 1.

Table 1 Parameter combinations used to predict sepsis

| First leukocyte parameter | Second leukocyte parameter | First leukocyte parameter | Second leukocyte parameter |
|---|---|---|---|
| D_Mon_SS_P | D_Mon_SS_W | D_Neu_FL_P | D_Mon_FL_W |
| D_Mon_SS_W | D_Mon_FL_P | D_Mon_FL_P | D_Mon_FL_W |
| D_Mon_SS_W | D_Mon_FS_P | D_Neu_SS_W | D_Mon_FS_P |
| D_Neu_FS_W | D_Mon_SS_W | D_Neu_FL_P | D_Neu_FL_W |
| D_Mon_SS_W | D_Mon_FL_W | D_Neu_FS_W | D_Mon_FL_W |
| D_Mon_SS_W | D_Mon_FS_W | D_Neu_SS_P | D_Mon_SS_P |
| D_Neu_SS_W | D_Mon_SS_W | D_Neu_FL_W | D_Neu_FS_W |
| D_Neu_FS_P | D_Mon_SS_W | D_Neu_SS_W | D_Mon_FL_P |
| D_Neu_FL_P | D_Mon_SS_W | D_Neu_FL_P | D_Mon_SS_P |
| D_Neu_SS_P | D_Mon_SS_W | D_Neu_FS_W | D_Mon_SS_P |
| D_Neu_FL_W | D_Mon_SS_W | D_Mon_SS_P | D_Mon_FS_W |

(continued)

| First leukocyte parameter | Second leukocyte parameter | First leukocyte parameter | Second leukocyte parameter |
|---|---|---|---|
| D_Neu_FL_W | D_Mon_FS_W | D_Neu_SS_P | D_Mon_FL_P |
| D_Neu_SS_W | D_Neu_FL_W | D_Neu_SS_P | D_Mon_FS_P |
| D_Neu_FS_P | D_Mon_FL_W | D_Neu_FS_P | D_Mon_SS_P |
| D_Neu_SS_W | D_Mon_FL_W | D_Neu_SS_W | D_Neu_FL_P |
| D_Neu_FL_W | D_Mon_FL_W | D_Mon_SS_P | D_Mon_FL_P |
| D_Mon_SS_P | D_Mon_FL_W | D_Mon_SS_P | D_Mon_FS_P |
| D_Neu_SS_P | D_Neu_FL_W | D_Neu_SS_P | D_Neu_FL_P |
| D_Neu_SS_W | D_Mon_SS_P | D_Neu_SS_W | D_Neu_FS_P |
| D_Neu_FL_W | D_Mon_SS_P | D_Neu_SS_W | D_Neu_FS_W |
| D_Mon_FL_W | D_Mon_FS_P | D_Neu_SS_P | D_Neu_SS_W |
| D_Neu_SS_P | D_Mon_FL_W | D_Mon_FL_P | D_Mon_FS_W |
| D_Neu_SS_W | D_Mon_FS_W | D_Neu_FL_P | D_Mon_FS_P |
| D_Neu_FL_W | D_Mon_FS_P | D_Neu_FL_P | D_Mon_FL_P |
| D_Neu_SS_P | D_Mon_FS_W | D_Neu_SS_P | D_Neu_FS_P |
| D_Mon_FL_W | D_Mon_FS_W | D_Neu_SS_P | D_Neu_FS_W |
| D_Neu_FL_W | D_Mon_FL_P | D_Neu_FL_P | D_Neu_FS_W |
| D_Neu_FL_P | D_Mon_FS_W | D_Neu_FS_P | D_Mon_FS_W |
| D_Neu_FL_W | D_Neu_FS_P | | |

In some embodiments, the processor 140 may be further configured to: output prompt information indicating that the infection marker parameter is abnormal when a value of the infection marker parameter is outside a preset range. For example, when the value of the infection marker parameter is abnormally elevated, an upward pointing arrow may be output to indicate the abnormal elevation.

Alternatively, processor 140 may be further configured to output the preset range.

Some embodiments for further ensuring diagnostic reliability based on the infection marker parameter will be described next, although it will be understood that embodiments of the disclosure are not limited thereto.

In order to avoid the leukocyte parameter for calculating the infection marker parameter itself interfering with diagnostic reliability, in some embodiments, the processor 140 may be further configured to either skip outputting the value of the infection marker parameter (i.e., screen the value of the infection marker parameter) or output the value of the infection marker parameter and simultaneously output prompt information indicating that the value of the infection marker parameter is unreliable when a preset characteristic parameter of the at least one leukocyte particle population satisfies a preset condition.

In one specific example, the processor 140 may be configured to skip outputting the value of the infection marker parameter, or output the value of the infection marker parameter and simultaneously output prompt information indicating that the value of the infection marker parameter is unreliable, when a total number of particles of the at least one leukocyte particle population is less than a preset threshold.

That is to say, when the total number of particles in the leukocyte particle population is less than the preset threshold, that is, the number of particles in the leukocyte particle population is small, and the amount of information characterized by the particles is limited, the calculation result of the infection marker parameter may not be reliable. For example, as shown in FIG. 7(a), the total number of particles of the leukocyte population in the test sample is too low, which may cause the infection marker parameter calculated from the leukocyte parameter of the leukocyte population to be unreliable.

Herein, for example, it is possible to determine whether the preset characteristic parameter of the leukocyte particle population is abnormal, for example, whether the total number of particles of the leukocyte particle population is lower than the preset threshold value, based on the optical information of the test sample.

In other examples, the processor 140 may be configured to skip outputting the value of the infection marker parameter, or output the value of the infection marker parameter and simultaneously output prompt information indicating that the value of the infection marker parameter is unreliable, when the at least one leukocyte particle population overlaps with another particle population.

For example, as shown in FIG. 7(b), there is an overlap between the monocyte population and the lymphocyte population in the test sample, which may lead to unreliable calculation of the infection marker parameter from the leukocyte parameters of the monocyte population or lymphocyte population.

Herein, for example, it is possible to determine whether the leukocyte particle population used overlaps with another particle population based on the optical information of the test sample.

In addition, disease status of the subject, as well as abnormal cells in blood of the subject, may also affect diagnostic efficacy of the infection marker parameter. To this end, processor 140 may be further configured to: determine the reliability of the infection marker parameter based on whether the subject has a specific disease and/or based on the presence of predefined types of abnormal cells (e.g., blast cells, abnormal lymphocytes, naive granulocytes, etc.) in the blood sample to be tested.

In some specific examples, the processor 140 may be configured to skip outputting the value of the infection marker parameter, or output the value of the infection marker parameter and simultaneously output prompt information indicating that the value of the infection marker parameter is unreliable, when the subject suffers from a hematological disorder or there are abnormal cells, especially blast cells, in the blood sample to be tested. It will be appreciated that an abnormal hemogram of a subject with a hematological disorder results in an unreliable diagnosis based on the infection marker parameter.

Processor 140 may, for example, obtain whether the subject suffers from a hematological disorder based on the subject's identity information.

In some embodiments, processor 140 may be configured to determine whether abnormal cells, in particular blast cells, are present in the blood sample to be tested based on the optical information.

In some embodiments, the processor 140 may further be configured to perform data processing, such as de-noising (impurity particles) interference (as shown in FIG. 7 (c)) on the leukocyte parameter used prior to calculating the infection marker parameter, in order to calculate the infection marker parameter more accurately, e.g., to avoid signal variations caused by different instruments, or different reagents.

In some embodiment, the processor 140 is further configured to: output prompt information indicating that the subject is likely to progress to sepsis within the certain period of time, when it is determined from the infection marker parameter that the subject is likely to progress to sepsis within a certain period of time starting from when the blood sample to be tested is collected. For example, when the value of the infection marker parameter is greater than a preset threshold, it is determined that the subject is likely to progress to sepsis within a certain period of time starting from when the blood sample to be tested is collected. The preset threshold may be determined based on a specific parameter or combination of parameters and the blood cell analyzer.

Further, the processor 140 may be configured to output the prompt information to a display device for display. The display device herein may be the display device 150 of the blood cell analyzer 100, or another display device in communication with the processor 140. For example, the processor 140 may output the prompt information to a display device on the user (doctor) side through the hospital information management system.

Similarly, the processor 140 may be further configured to skip outputting prompt information indicating that the subject is likely to progress to sepsis within the certain period of time, or output the prompt information and output additional information indicating that the prompt information is unreliable, when a preset characteristic parameter of the at least one leukocyte particle population satisfies a preset condition, such as when a total number of particles of the at least one leukocyte particle population is less than a preset threshold and/or when the at least one leukocyte particle population overlaps with another particle population.

Alternatively or additionally, the processor 140 may be further configured to skip outputting prompt information indicating that the subject is likely to progress to sepsis within the certain period of time, or output the prompt information and output additional information indicating that the prompt information is unreliable, when the subject suffers from a hematological disorder or there are abnormal cells, especially blast cells, in the blood sample to be tested, such as when it is determined that there are abnormal cells, especially blast cells, in the blood sample to be tested based on the optical information.

The manner in which the processor 140 assigns a priority for each set of infection marker parameters will be described below in conjunction with some of following embodiments. In some embodiments, the processor 140 may be further configured to: assign a priority for each set of infection marker parameters based on at least one of infection diagnostic efficacy, parametric stability and parametric limitations.

Preferably herein, the processor 140 may be further configured to: assign a priority for each set of infection marker parameters based at least on the infection diagnostic efficacy. For example, the processor 140 may assign a priority for each set of infection marker parameters based only on infection diagnostic efficacy. For still another example, the processor 140 may assign a priority for each set of infection marker parameters based on infection diagnostic efficacy and parametric stability; For yet another example, the processor 140 may assign a priority for each set of infection marker parameters based on infection diagnostic efficacy, parametric stability, and parametric limitations.

In some embodiments, the set of infection marker parameters of the disclosure may be used for the evaluation of a variety of infection statuses, for example for early prediction of sepsis. Accordingly, the infection diagnostic efficacy includes diagnostic efficacy for early prediction of sepsis. For example, when the set of infection marker parameters of the disclosure is set only for evaluation of one infection status, for example, only for early prediction of sepsis, each set of infection marker parameters may be assigned a priority based on diagnostic efficacy for the evaluation of infection status, for example, early prediction of sepsis.

As some implementations, the processor 140 may be further configured to: assign a priority for each set of infection marker parameters according to an area ROC_AUC enclosed by ROC curve of each set of infection marker parameters and the horizontal coordinate axis, wherein the larger the ROC_AUC, the higher the priority of the corresponding set of infection marker parameters. In this case, the ROC curve is a receiver operating characteristic curve drawn with the true positive rate as the ordinate and the false positive rate as the abscissa. The ROC_AUC of each set of infection marker parameters may reflect the infection diagnostic efficacy of the set of infection marker parameters.

In some embodiments, the parametric stability includes at least one of numerical repeatability, aging stability, temperature stability and inter-machine consistency. The numerical repeatability refers to numerical consistency of a set of infection marker parameters used when a same test blood sample is tested for multiple times using a same instrument in a short period of time under a same environment; the aging stability refers to numerical stability of a set of infection marker parameters used when a same test blood sample is tested using a same instrument at different time points under a same environment; the temperature stability refers to numerical stability of a set of infection marker parameters used when a same test blood sample is tested using a same instrument under different temperature environments; and the inter-machine consistency refers to numerical consistency of a set of infection marker parameters used when a same test blood sample is tested using different instruments under a same environment.

In some examples, if a same test blood sample is tested for multiple times using a same instrument in a short period of time under a same environment, the higher the numerical consistency of the set of infection marker parameters used, that is, the higher the numerical repeatability, the higher the priority of the set of infection marker parameters.

Alternatively or additionally, if a same test blood sample is tested using a same instrument at different time points under a same environment, the higher the numerical stability of the set of infection marker parameters used (that is, the smaller the numerical fluctuation degree), that is, the higher the aging stability, the higher the priority of the set of infection marker parameters.

Alternatively or additionally, if a same test blood sample is tested using a same instrument under different temperature environments, the higher the numerical stability of the set of infection marker parameters used (that is, the smaller the numerical fluctuation degree), that is, the higher the temperature stability, the higher the priority of the set of infection marker parameters.

Alternatively or additionally, when a same test blood sample is tested using different instruments under a same environment, the higher the numerical consistency of the set of infection marker parameters used, that is, the higher the inter-machine consistency, the higher the priority of the set of infection marker parameters.

In some embodiments, the parametric limitation refers to the range of subjects to which the infection marker parameter is applicable. In some examples, if the range of subjects to which the set of infection marker parameters is applicable is larger, it means that the parametric limitation of the set of infection marker parameters is smaller, and correspondingly, the priority of the set of infection marker parameters is higher.

In some embodiments, the priorities of the plurality of sets of infection marker parameters obtained by the processor 140 are preset, for example, based on at least one of the infection diagnostic efficacy, the parametric stability and the parametric limitations. Here, the processor 140 may assign a priority for each set of infection marker parameters based on the preset. For example, the priorities of the plurality of sets of infection marker parameters may be stored in a memory in advance, and the processor 140 may invoke the priorities of the pluralities of sets of infection marker parameters from the memory.

Next, the manner in which the processor 140 calculates a credibility of a set of infection marker parameters will be further described in conjunction with some of following embodiments.

The inventors of the disclosure have found through research that there may be abnormal classification results and/or abnormal cells in the blood sample of the subject, resulting in unreliability of the set of infection marker parameters used. Accordingly, the blood analyzer provided in the disclosure can calculate respective credibility for the obtained plurality of sets of infection marker parameters in order to screen out a more reliable set of infection marker parameters from the plurality of sets of infection marker parameters based on respective priority and credibility of each set of infection marker parameters.

In some embodiments, the processor 140 may be configured to calculate a credibility for each set of infection marker parameters as follows:

calculating respective credibility of each set of infection marker parameters according to a classification result of at least one target particle population used to obtain said set of infection marker parameters and/or according to abnormal cells in the blood sample to be tested.

In some embodiments, the classification result may include at least one of a count value of the target particle population, a count value percentage of the target particle population to another particle population, and a degree of overlap (also referred to as a degree of adhesion) between the target particle population and its adjacent particle population. For example, the degree of overlap between the target particle population and its adjacent particle population may be determined by the distance between the center of gravity of the target particle population and the center of gravity of its adjacent particle population. For example, if a total number of particles of the target particle population, that is, the count value, is less than a preset threshold, that is, the particles of the target particle population are few, and the amount of information characterized by the particles is limited, at this time, the set of infection marker parameters obtained through relevant parameters of the target particle population may be unreliable, so the credibility of the set of infection marker parameters is relatively low.

Next, the manner in which the processor 140 screens a set of infection marker parameters will be further described in conjunction with some embodiments.

In an embodiment of the disclosure, the processor 140 may be configured to calculate respective credibility for all of the sets of infection marker parameters in the plurality of sets of infection marker parameters at a time, and then select at least one set of infection marker parameters from all of the sets of infection marker parameters based on the respective priority and credibility of all of the sets of infection marker parameters and output their parameter value.

In other embodiments, the processor 140 may be configured to perform following steps to screen the set of infection marker parameters and output its parameter values:

obtaining a plurality of parameters of at least one target particle population in the test sample from the optical information;
obtaining a plurality of sets of infection marker parameters for evaluating an infection status of the subject from the plurality of parameters;
according to respective priority of the plurality of sets of infection marker parameters, successively calculating respective credibility of the plurality of sets of infection marker parameters and determining whether the respective credibility reaches a corresponding credibility threshold;
when the respective credibility of the current set of infection marker parameters reaches the corresponding credibility threshold, outputting the parameter value of said set of infection marker parameters and stopping the calculation and determination.

In some embodiments, the processor 140 may be further configured to output an alarm prompt, when the parameter value of the selected set of infection marker parameters is greater than an infection positive threshold.

Herein, for example, each set of infection marker parameters may be normalized to ensure that infection positivity thresholds of respective infection marker parameters are consistent.

In other embodiments, the processor 140 may be further configured to obtain a plurality of parameters of at least one target particle population in the test sample from the optical information,

obtain a plurality of sets of infection marker parameters for evaluating an infection status of the subject from the plurality of parameters,
calculate a credibility of each set of infection marker parameters of the plurality of sets of infection marker parameters,
select at least one set of infection marker parameters from the plurality of sets of infection marker parameters based on respective credibility of the plurality of sets of infection marker parameters and output their parameter values.

In some embodiments, the processor may be further configured to:
for each set of infection marker parameters, calculate respective credibility of said set of infection marker parameters based on a classification result of at least one target particle population used to obtain said set of infection marker parameters and/or based on abnormal cells in the blood sample to be tested.

The classification result may include, for example, at least one of a count value of the target particle population, a count value percentage of the target particle population to another particle population, and a degree of overlap between the target particle population and its adjacent particle population.

Further, the processor is further configured to:
output an alarm prompt, when the parameter value of the selected set of infection marker parameters is greater than the infection positive threshold.

In other embodiments, the processor 140 may be further configured to determine, based on the optical information, whether the blood sample to be tested has an abnormality that affect the evaluation of infection status; when it is determined that there is an abnormality in the blood sample to be tested that affects the evaluation of infection status, obtain an infection marker parameter unaffected by the abnormality and used to evaluate the infection status of the subject from the optical information.

In one example, if it is determined that there is an abnormal classification result affecting the evaluation of infection status in the blood sample to be tested, for example, there is an overlap between the monocyte population and the neutrophil population in the blood sample to be tested, a plurality of parameters of another cell population (such as the lymphocyte population) other than the monocyte population and the neutrophil population can be obtained from the optical information, and an infection marker parameter for evaluating the infection status of the subject can be obtained from the plurality of parameters of the another cell population.

In another example, if it is determined that there are abnormal cells, such as blast cells, affecting the evaluation of infection status in the blood sample to be tested, a plurality of parameters of another cell population other than the cell population affected by the blast cells can be obtained from the optical information, and an infection marker parameter for evaluating the infection status of the subject can be obtained from the plurality of parameters of the another cell population.

Next, the manner in which the processor 140 controls a retest will be further described in conjunction with some embodiments.

In some embodiments, the processor may be further configured to:

obtain a leukocyte count of the subject before obtaining at least one leukocyte parameter of at least one leukocyte particle population in the test sample from the optical information, and output a retest instruction to retest the blood sample of the subject when the leukocyte count is less than a preset threshold, wherein a measurement amount of the sample to be retested based on the retest instruction is greater than a measurement amount of the sample to be tested to obtain the optical information; and

obtain at least another leukocyte parameter of at least another leukocyte particle population from optical information measured based on the retest instruction, and obtain an infection marker parameter for determining that the subject is likely to progress to sepsis within a certain period of time based on the at least another leukocyte parameter.

The disclosure further provides yet another blood analyzer including a measurement device and a controller, wherein

the measurement device is configured to mix a blood sample of a subject to be tested, a hemolytic agent and a staining agent to prepare a test sample and perform optical measurement on the test sample to obtain optical information of the test sample;

the controller is configured to: receive a mode setting instruction, and when the mode setting instruction indicates that a blood routine test mode is selected, control the measurement device to perform optical measurement on a first measurement amount of the test sample to obtain optical information of the test sample, and obtain and output blood routine parameters of the test sample based on said optical information; when the mode setting instruction indicates that a sepsis test mode is selected, control the measurement device to perform optical measurement on a second measurement amount of the test sample, the second measurement amount being greater than the first measurement amount, to obtain optical information of the test sample, calculate at least one leukocyte parameter of at least one leukocyte particle population in the test sample from said optical information, obtain an infection marker parameter based on the at least one leukocyte parameter, and output the infection marker parameter, wherein the infection marker parameter is used for predicting whether the subject is likely to progress to sepsis within a certain period of time starting from when the blood sample to be tested is collected.

To this end, it is possible to control the sample analyzer to perform a retest action when the leukocyte count in the sample is less than a preset threshold, which results in unreliable test parameter results, so as to obtain more accurate infection marker parameters for predicting whether the subject is likely to progress to sepsis within a certain period of time starting from when the blood sample to be tested is collected.

Embodiments of the disclosure also provide a method 200 for predicting whether a subject is likely to progress to sepsis in the future. As shown in FIG. 8, the method 200 includes the steps of:

S210: obtaining a blood sample to be tested from the subject;

S220: preparing a test sample containing a part of the blood sample to be tested, a hemolytic agent, and a staining agent for leukocyte classification;

S230: passing particles in the test sample through an optical detection region irradiated with light one by one, to obtain optical information generated by the particles in the test sample after being irradiated with light;

S240: calculating at least one leukocyte parameter of at least one leukocyte subpopulation in the test sample from the optical information;

S250: obtaining an infection marker parameter based on the at least one leukocyte parameter; and
S260: predicting based on the infection marker parameter whether the subject is likely to progress to sepsis within a certain period of time starting from when the blood sample to be tested is collected.

The method 200 provided in the embodiments of the disclosure is implemented, in particular, by the blood cell analyzer 100 described above in the embodiments of the disclosure.
Further, in the method 200, the at least one leukocyte parameter may include one or more of cell characteristic parameters of monocyte population, neutrophil population and lymphocyte population in the test sample. Preferably the at least one leukocyte parameter may include one or more of cell characteristic parameters of monocyte population and neutrophil population in the test sample.
Further, in the method 200, the at least one leukocyte parameter may include one or more of following parameters: a forward scatter intensity distribution width, a forward scatter intensity distribution center of gravity, a forward scatter intensity distribution variation coefficient, a side scatter intensity distribution width, a side scatter intensity distribution center of gravity, a side scatter intensity distribution coefficient of variation, a fluorescence intensity distribution width, a fluorescence intensity distribution center of gravity, a fluorescence intensity distribution coefficient of variation of the leukocyte particle population, and an area of a distribution region of the leukocyte particle population in a two-dimensional scattergram generated by two light intensities selected from forward scatter intensity, side scatter intensity and fluorescence intensity, and a volume of a distribution region of the leukocyte particle population in a three-dimensional scattergram generated by forward scatter intensity, side scatter intensity and fluorescence intensity.
Preferably, the at least one leukocyte parameter may include one or more of following parameters: a forward scatter intensity distribution width, a forward scatter intensity distribution center of gravity, a forward scatter intensity distribution variation coefficient, a side scatter intensity distribution width, a side scatter intensity distribution center of gravity, a side scatter intensity distribution coefficient of variation, a fluorescence intensity distribution width, a fluorescence intensity distribution center of gravity, a fluorescence intensity distribution coefficient of variation of monocyte population in the test sample, and an area or volume of a distribution region of monocyte population in a two-dimensional scattergram generated by two light intensities selected from forward scatter intensity, side scatter intensity and fluorescence intensity, and a volume of a distribution region of monocyte population in a three-dimensional scattergram generated by forward scatter intensity, side scatter intensity and fluorescence intensity; and a forward scatter intensity distribution width, a forward scatter intensity distribution center of gravity, a forward scatter intensity distribution variation coefficient, a side scatter intensity distribution width, a side scatter intensity distribution center of gravity, a side scatter intensity distribution coefficient of variation, a fluorescence intensity distribution width, a fluorescence intensity distribution center of gravity, a fluorescence intensity distribution coefficient of variation of neutrophil population in the test sample, and an area of a distribution region of neutrophil population in a two-dimensional scatter plot generated by two light intensities selected from forward scatter intensity, side scatter intensity and fluorescence intensity, and a volume of a distribution region of neutrophil population in a three-dimensional scatter plot generated by forward scatter intensity, side scatter intensity and fluorescence intensity.
Further, steps S240 and S250 may include:

calculating at least one first leukocyte parameter of a first leukocyte particle population in the test sample and at least one second leukocyte parameter of a second leukocyte particle population in the test sample from the optical information, preferably, the first leukocyte particle population being monocyte population and the second leukocyte particle population being neutrophil population; and
calculating the infection marker parameter based on the at least one first leukocyte parameter and the at least one second leukocyte parameter, in particular by using a linear function.

Further, in the method 200, the certain period of time is not greater than 48 hours, preferably not greater than 24 hours.
Alternatively, steps S240 and S250 may include:

obtaining at least two leukocyte parameters of one leukocyte particle population in the test sample from the optical information; and
calculating the infection marker parameter based on the at least two leukocyte parameters, in particular by using a linear function.

Further, the method 200 may further include: outputting prompt information indicating that the infection marker parameter is abnormal when a value of the infection marker parameter is outside the preset range.
Further, the method 200 may further include:
skipping outputting a value of the infection marker parameter, or outputting a value of the infection marker parameter and simultaneously outputting prompt information indicating that the value of the infection marker parameter is unreliable, when a preset characteristic parameter of at least one leukocyte particle population in the test sample satisfies a preset condition, such as when a total number of particles of at least one leukocyte particle population in the test sample is less than a preset threshold and/or when the at least one leukocyte particle population overlaps with another particle population.

Alternatively or additionally, the method 200 may further include: skipping outputting a value of the infection marker parameter, or outputting a value of the infection marker parameter and simultaneously outputting prompt information indicating that the value of the infection marker parameter is unreliable, when the subject suffers from a hematological disorder or there are abnormal cells, especially blast cells, in the blood sample to be tested, such as when it is determined that there are abnormal cells, especially blast cells, in the blood sample to be tested based on the optical information.

Alternatively, the method 200 further includes: determining whether the subject is likely to progress to sepsis within a certain period of time starting from when the blood sample to be tested is collected according to the infection marker parameter, and outputting prompt information indicating that the subject is likely to progress to sepsis within the certain period of time starting from when the blood sample to be tested is collected when it is determined that the subject is likely to progress to sepsis within the certain period of time starting from when the blood sample to be tested is collected according to the infection marker parameter.

For further embodiments and advantages of the method 200 provided by the embodiments of the disclosure, reference may be made to the above description of the blood cell analyzer 100 provided by the embodiments of the disclosure, in particular the description of the method and steps performed by the processor 140, which will not be described here in detail.

Embodiments of the disclosure further provide the use of an infection marker parameter in predicting whether a subject is likely to progress to sepsis in the future, wherein the infection marker parameter is obtained by:

obtaining at least one leukocyte parameter of at least one leukocyte particle population obtained by flow cytometry detection of a test sample containing a part of a blood sample to be tested from the subject, a hemolytic agent, and a staining agent for leukocyte classification; and
obtaining an infection marker parameter based on the at least one leukocyte parameter.

For further embodiments and advantages of the use of the infection marker parameters provided by the embodiments of the disclosure in evaluating the infection status of a subject, reference may be made to the above description of the blood cell analyzer 100 provided by the embodiments of the disclosure, and in particular the description of the methods and steps performed by the processor 140, which will not be repeated herein.

Next, the disclosure and its advantages will be further explained with specific examples.

True positive rate%, false positive rate%, true negative rate%, and false negative rate% of embodiments of the disclosure are calculated by the following equations:

$$\text{True positive rate\%} = \text{TP}/\ (\text{TP} + \text{FN}) \times 100\%,$$

$$\text{True negative rate\%} = \text{TN}/\ (\text{FP} + \text{TN}) \times 100\%,$$

$$\text{False positive rate\%} = 1\text{-true negative rate\%},$$

and

$$\text{False negative rate\%} = 1\text{-true positive rate\%},$$

wherein TP is the number of true positive individuals, FP is the number of false positive individuals, TN is the number of true negative individuals, and FN is the number of false negative individuals.

For early prediction of sepsis, blood samples from 152 donors were tested using BC-6800 Plus blood cell analyzer produced by SHENZHEN MINDRAY BIOMEDICAL ELECTRONICS CO., LTD. in accordance with the method provided in the embodiments of the disclosure. In addition, clinical tests were performed on the blood samples from the 152 donors after 1 day. The results showed that among the above-mentioned blood samples, 87 positive samples with sepsis and 65 negative samples (the donors of the negative samples did not develop into sepsis) were clinically diagnosed after 1 day.

Inclusion criteria: adult ICU patients with or without acute infection.

Exclusion criteria: pregnant people, myelosuppressed people on chemotherapy, people on immunosuppressant treatment, patients with hematologic diseases.

For the donors of the sepsis samples: they have a suspicious or definite infection site, a positive laboratory culture result, and organ failure; they have suspicious or confirmed acute infection, and SOFA score ≥ 2 , where the suspected infection has any of the following (1) - (3) and has no deterministic results for (4); or has any one of the following (1) - (3) and (5).

(1) Acute (within 72 hours) fever or hypothermia;
(2) Increased or decreased total number of leukocytes;
(3) Increased CRP and IL-6;
(4) Increased PCT, SAA and HBP;
(5) Presence of suspicious infection sites.

The SOFA scoring criteria are shown in the table below:

Table SOFA score calculation method

| Organ | Variable | Score 0 | Score 1 | Score 2 | Score 3 | Score 4 |
|---|---|---|---|---|---|---|
| Respiratory system | $PaO_2/PiO_2$/mmHg | ≥400 | <400 | <300 | <200 | <100 |
| Blood system | PLT/(×$10^9$·$L^{-1}$) | ≥150 | <150 | <100 | <60 | <20 |
| Liver | Bilirubin/(mg·$dL^{-1}$) | <1.2 | 1.2~1.9 | 2.0~3.4 | 3.5~4.9 | ≥5.0 |
| Central nervous system | Glasgow Score | 15 | 13~14 | 10~12 | 6~9 | <6 |
| Kidney | Creatinine/(mg·$dL^{-1}$) | <1.2 | 1.2~1.9 | 2.0~3.4 | 3.5~4.9 | ≥5.0 |
| | Urine volume/(mL·$d^{-1}$) | ≥500 | - | - | <500 | <200 |
| Circulation | Mean arterial pressure/mmHg | ≥70 | <70 | - | - | - |
| | Dopamine /[μg·$kg^{-1}$·$min^{-1}$] | | | ≤5 | >5 | >15 |
| | Dobutamine | | | Any dose | | |
| | Epinephrine/[μg·$kg^{-1}$·$min^{-1}$] | - | - | - | ≤0.1 | >0.1 |
| | Norepinephrine /[μg·$kg^{-1}$· $min^{-1}$] | - | - | - | ≤0.1 | >0.1 |

Note .1 mmHg=0.133 kPa.

Table 2 shows diagnostic efficacy of using a single leukocyte parameter as an infection marker parameter, and Table 3 shows diagnostic efficacy of using a combination of two parameters (a combination of a first leukocyte parameter and a second leukocyte parameter) as an infection marker parameter. FIG. 9 shows ROC curve of using a single leukocyte parameter as an infection marker parameter, and FIG. 10 shows ROC curve of using a combination of two leukocyte parameters as an infection marker parameter.

An infection marker parameter is calculated by the function Y = A * X1 + B* X2 + C based on the first leukocyte parameter and the second leukocyte parameter in Table 3, wherein Y represents the infection marker parameter, X1 represents the first leukocyte parameter, X2 represents the second leukocyte parameter, and A, B, and C are constants.

Table 2 Efficacy of single parameter for predicting sepsis

| Single parameter | ROC_ AUC | Determina tion threshold | False positive rate% | True positive rate% | True negative rate% | False negative rate% |
|---|---|---|---|---|---|---|
| D_Mon_ SS_W | 0.6771 | >89.388 | 26.2 | 60.9 | 73.8 | 39.1 |
| D_Mon_ FL_W | 0.6343 | >453.7485 | 16.9 | 43.7 | 83.1 | 56.3 |
| D_Neu_ FL_W | 0.6334 | >205.4925 | 47.7 | 72.4 | 52.3 | 27.6 |

Table 3 Efficacy of two parameters for predicting sepsis

| First leukocyte parameter | First leukocyte parameter | ROC_ AUC | Determin ation threshold | False positive rate% | True posit ive rate % | True negat ive rate % | False negat ive rate % | A | B | c |
|---|---|---|---|---|---|---|---|---|---|---|
| D Mon S S_P | D_Mon_S S_W | 0.6858 | >0.2396 | 26.2 | 62.1 | 73.8 | 37.9 | -0.0043 | 0.039459 | -2.33948 |
| D_Mon_S S_W | D_Mon_F L P | 0.684 | >0.2718 | 29.2 | 60.9 | 70.8 | 39.1 | 0.041206 | -0.00094 | -2.5351 |
| D_Mon_S S_W | D_Mon_F S_P | 0.684 | >0.2826 | 26.2 | 60.9 | 73.8 | 39.1 | 0.045283 | -0.00234 | -0.78827 |
| D_Neu_F S_W | D_Mon_S S_W | 0.6835 | >0.2231 | 33.8 | 64.4 | 66.2 | 35.6 | -0.0035 | 0.039183 | -1.31617 |
| D_Mon_S S_W | D_Mon_F L_W | 0.6798 | >0.1834 | 30.8 | 60.9 | 69.2 | 39.1 | 0.030048 | 0.00471 | -4.48052 |
| D_Mon_S S_W | D_Mon_F S_W | 0.6796 | >0.298 | 23.1 | 58.6 | 76.9 | 41.4 | 0.038609 | -0.00129 | -2.73487 |
| D_Neu_S S_W | D_Mon_S S_W | 0.6792 | >0.1682 | 32.3 | 66.7 | 67.7 | 33.3 | 0.005358 | 0.031244 | -3.97398 |
| D_Neu_F S_P | D_Mon_S S_W | 0.679 | >0.1919 | 30.8 | 63.2 | 69.2 | 36.8 | 0.001846 | 0.034412 | -6.08306 |
| D_Neu_F L P | D_Mon_S S_W | 0.6764 | >0.2529 | 26.2 | 58.6 | 73.8 | 41.4 | 0.003008 | 0.03073 | -3.9261 |
| D_Neu_S S_P | D_Mon_S S_W | 0.675 | >0.2031 | 30.8 | 65.5 | 69.2 | 34.5 | 0.003787 | 0.031643 | -3.98386 |
| D_Neu_F L_W | D_Mon_S S_W | 0.6732 | >0.0652 | 41.5 | 72.4 | 58.5 | 27.6 | 0.008524 | 0.025486 | -3.89268 |
| D_Neu_F L_W | D_Mon_F S_W | 0.6568 | >0.1394 | 40 | 71.3 | 60 | 28.7 | 0.014875 | 0.00425 | -4.54817 |
| D_Neu_S S_W | D_Neu_F L_W | 0.6534 | >0.2399 | 35.4 | 62.1 | 64.6 | 37.9 | 0.005193 | 0.013571 | -4.07506 |
| D_Neu_F S_P | D_Mon_F L_W | 0.6531 | >0.0883 | 43.1 | 77 | 56.9 | 23 | 0.002811 | 0.00713 | -7.7575 |
| D_Neu_S S_W | D_Mon_F L_W | 0.6527 | >0.215 | 38.5 | 64.4 | 61.5 | 35.6 | 0.009553 | 0.00664 | -5.14314 |
| D_Neu_F L W | D_Mon_F L W | 0.6499 | >0.2368 | 36.9 | 59.8 | 63.1 | 40.2 | 0.013617 | 0.00536 | -5.02647 |
| D_Mon_S S_P | D_Mon_F L_W | 0.6495 | >0.2578 | 41.5 | 60.9 | 58.5 | 39.1 | 0.021027 | 0.006618 | -7.22136 |
| D_Neu_S S_P | D_Neu_F L_W | 0.6492 | >0.2066 | 36.9 | 62.1 | 63.1 | 37.9 | 0.004323 | 0.01372 | -4.32426 |
| D_Neu_S S_W | D_Mon_S S_P | 0.6474 | >0.3018 | 32.3 | 56.3 | 67.7 | 43.7 | 0.00699 | 0.017315 | -5.39448 |
| D_Neu_F L_W | D_Mon_S S_P | 0.6458 | >0.1109 | 43.1 | 69 | 56.9 | 31 | 0.012707 | 0.01089 | -4.9011 |
| D_Mon_F L_W | D_Mon_F S_P | 0.6458 | >0.3187 | 32.3 | 56.3 | 67.7 | 43.7 | 0.007128 | 0.003188 | -6.92835 |
| D_Neu_S S_P | D_Mon_F L_W | 0.6447 | >0.3394 | 30.8 | 54 | 69.2 | 46 | 0.009053 | 0.006601 | -5.93211 |
| D_Neu_S S_W | D_Mon_F S_W | 0.6437 | >0.2161 | 36.9 | 60.9 | 63.1 | 39.1 | 0.008957 | 0.004558 | -3.78741 |
| D_Neu_F L_W | D_Mon_F S_P | 0.6405 | >0.0673 | 44.6 | 70.1 | 55.4 | 29.9 | 0.01474 | 0.001199 | -4.49666 |

As can be seen from Tables 2 to 3 and FIGS. 9 to 10, the infection marker parameter provided in the disclosure can be used to predict risk of sepsis effectively one day in advance.

The features or combinations thereof mentioned above in the description, accompanying drawings, and claims can be combined with each other arbitrarily or used separately as long as they are meaningful within the scope of the disclosure and do not contradict each other.

The foregoing description merely relates to the preferred embodiments of the disclosure, and is not intended to limit the scope of patent of the disclosure. All equivalent variations made by using the content of the specification and the accompanying drawings of the disclosure from the concept of the disclosure, or the direct/indirect applications of the contents in other related technical fields all fall within the scope of patent protection of the disclosure.

## Claims

1. A blood cell analyzer, **characterized in that** the blood cell analyzer comprises:

    a sample aspiration device configured to aspirate a blood sample of a subject to be tested;
    a sample preparation device configured to prepare a test sample containing a part of the blood sample to be tested, a hemolytic agent, and a staining agent for leukocyte classification;
    an optical detection device comprising a flow cell, a light source and an optical detector, the flow cell being configured to allow the test sample to pass through, the light source being configured to irradiate with light the test sample passing through the flow cell, and the optical detector being configured to detect optical information generated by the test sample under irradiation when passing through the flow cell; and
    a processor configured to:

        calculate at least one leukocyte parameter of at least one leukocyte particle population in the test sample from the optical information,
        obtain an infection marker parameter based on the at least one leukocyte parameter, and
        output the infection marker parameter, wherein the infection marker parameter is used for predicting whether the subject is likely to progress to sepsis within a certain period of time starting from when the blood sample to be tested is collected.

2. The blood cell analyzer of claim 1, **characterized in that**, the at least one leukocyte parameter comprises one or more of cell characteristic parameters of monocyte population, neutrophil population and lymphocyte population in the test sample; preferably, the at least one leukocyte parameter comprises one or more of cell characteristic parameters of monocyte population and neutrophil population in the test sample.

3. The blood cell analyzer of claim 1 or 2, **characterized in that**, the at least one leukocyte parameter comprises one or more of following parameters: a forward scatter intensity distribution width, a forward scatter intensity distribution center of gravity, a forward scatter intensity distribution variation coefficient, a side scatter intensity distribution width, a side scatter intensity distribution center of gravity, a side scatter intensity distribution coefficient of variation, a fluorescence intensity distribution width, a fluorescence intensity distribution center of gravity, a fluorescence intensity distribution coefficient of variation of the leukocyte particle population, and an area of a distribution region of the leukocyte particle population in a two-dimensional scattergram generated by two light intensities selected from forward scatter intensity, side scatter intensity and fluorescence intensity, and a volume of a distribution region of the leukocyte particle population in a three-dimensional scattergram generated by forward scatter intensity, side scatter intensity, and fluorescence intensity;
    preferably, the at least one leukocyte parameter comprises one or more of following parameters: a forward scatter intensity distribution width, a forward scatter intensity distribution center of gravity, a forward scatter intensity distribution variation coefficient, a side scatter intensity distribution width, a side scatter intensity distribution center of gravity, a side scatter intensity distribution coefficient of variation, a fluorescence intensity distribution width, a fluorescence intensity distribution center of gravity, a fluorescence intensity distribution coefficient of variation of monocyte population in the test sample, and an area of a distribution region of monocyte population in a two-dimensional scattergram generated by two light intensities selected from forward scatter intensity, side scatter intensity and fluorescence intensity, and a volume of a distribution region of monocyte population in a three-dimensional scattergram generated by forward scatter intensity, side scatter intensity and fluorescence intensity; and a forward scatter intensity distribution width, a forward scatter intensity distribution center of gravity, a forward scatter intensity distribution variation coefficient, a side scatter intensity distribution width, a side scatter intensity distribution center of gravity, a side scatter intensity distribution coefficient of variation, a fluorescence intensity distribution width, a fluorescence intensity distribution center of gravity, a fluorescence intensity distribution coefficient of variation of neutrophil population in the test sample, and an area of a distribution region of neutrophil population in a two-dimensional scattergram generated by two light intensities selected from forward scatter intensity, side scatter intensity and fluorescence intensity, and a volume of a distribution region of neutrophil population in a three-dimensional scattergram generated by forward scatter intensity, side scatter intensity and fluorescence intensity.

**4.** The blood cell analyzer of any one of claims 1 to 3, **characterized in that**, the processor is further configured to:

calculate at least one first leukocyte parameter of a first leukocyte particle population in the test sample and at least one second leukocyte parameter of a second leukocyte particle population in the test sample from the optical information, preferably, the first leukocyte particle population being monocyte population and the second leukocyte particle population being neutrophil population; and
calculate the infection marker parameter based on the at least one first leukocyte parameter and the at least one second leukocyte parameter, in particular, by a linear function.

**5.** The blood cell analyzer of any one of claims 1 to 3, **characterized in that**, the processor is further configured to:

calculate at least two leukocyte parameters of one leukocyte particle population in the test sample from the optical information; and
calculate the infection marker parameter based on the at least two leukocyte parameters, in particular, by a linear function.

**6.** The blood cell analyzer of any one of claims 1 to 5, **characterized in that**, the processor is further configured to: output prompt information indicating that the infection marker parameter is abnormal when a value of the infection marker parameter is outside a preset range.

**7.** The blood cell analyzer of any one of claims 1 to 6, **characterized in that**, the certain period of time is not greater than 48 hours, preferably not greater than 24 hours.

**8.** The blood cell analyzer of any one of claims 1 to 7, **characterized in that**, the processor is further configured to: skip outputting a value of the infection marker parameter, or output a value of the infection marker parameter and simultaneously output prompt information indicating that the value of the infection marker parameter is unreliable, when a preset characteristic parameter of the at least one leukocyte particle population meets a preset condition.

**9.** The blood cell analyzer of claim 8, **characterized in that**, the processor is further configured to: skip outputting a value of the infection marker parameter, or output a value of the infection marker parameter and simultaneously output prompt information indicating that the value of the infection marker parameter is unreliable, when a total number of particles of the at least one leukocyte particle population is less than a preset threshold and/or when the at least one leukocyte particle population overlaps with another population.

**10.** The blood cell analyzer of any one of claims 1 to 9, **characterized in that**, the processor is further configured to: skip outputting a value of the infection marker parameter, or output a value of the infection marker parameter and simultaneously output prompt information indicating that the value of the infection marker parameter is unreliable, when the subject suffers from a hematological disorder or there are abnormal cells, especially blast cells, in the blood sample to be tested, such as when it is determined that there are abnormal cells, especially blast cells, in the blood sample to be tested based on the optical information.

**11.** The blood cell analyzer of any one of claims 1 to 10, **characterized in that**, the processor is further configured to: output prompt information indicating that the subject is likely to progress to sepsis within the certain period of time, when it is determined from the infection marker parameter that the subject is likely to progress to sepsis within a certain period of time starting from when the blood sample to be tested is collected.

**12.** The blood cell analyzer of any one of claims 1 to 7, **characterized in that**, the processor is further configured to obtain a leukocyte count of the subject before calculating at least one leukocyte parameter of at least one leukocyte particle population in the test sample from the optical information, and output a retest instruction to retest the blood sample of the subject when the leukocyte count is less than a preset threshold, wherein a measurement amount of the sample to be retested based on the retest instruction is greater than a measurement amount of the sample to be tested to obtain the optical information; and

the processor is further configured to calculate at least another leukocyte parameter of at least another leukocyte particle population from optical information measured based on the retest instruction, and obtain an infection marker parameter for determining that the subject is likely to progress to sepsis within the certain period of time based on the at least another leukocyte parameter.

**13.** The blood cell analyzer of any one of claims 1 to 7, **characterized in that**, calculating at least one leukocyte parameter of at least one leukocyte particle population in the test sample from the optical information and obtaining an infection marker parameter based on the at least one leukocyte parameter by the processor comprises:

obtain a plurality of parameters of at least one leukocyte particle population in the test sample from the optical information;
obtain a plurality of sets of infection marker parameters for determining whether the subject has a severe infection from the plurality of parameters;
assign a priority for each set of infection marker parameters of the plurality of sets of infection marker parameters;
calculate a credibility of each set of infection marker parameters of the plurality of sets of infection marker parameters, select at least one set of infection marker parameters from the plurality of sets of infection marker parameters based on respective priority and credibility of the plurality of sets of infection marker parameters so as to obtain the infection marker parameter; or according to respective priority of the plurality of sets of infection marker parameters, successively calculate respective credibility of the plurality of sets of infection marker parameters and determine whether the respective credibility reaches a corresponding credibility threshold, and when the respective credibility of the current set of infection marker parameters reaches the corresponding credibility threshold, obtain the infection marker parameter based on said set of infection marker parameters and stop calculation and determination.

**14.** The blood cell analyzer of claim 13, **characterized in that**, the processor is further configured to:

calculate the credibility of each set of infection marker parameters of the plurality of sets of infection marker parameters, and determine whether the credibility of each set of infection marker parameters reaches a corresponding credibility threshold;
use the set(s) of infection marker parameters, whose respective credibility reaches the corresponding credibility threshold among the plurality of sets of infection marker parameters as candidate set(s) of infection marker parameters; and
select at least one candidate set of infection marker parameters from the candidate set(s) of infection marker parameters according to the respective priority of the candidate sets of infection marker parameters, preferably select a set of infection marker parameters with the highest priority, so as to obtain the infection marker parameter.

**15.** The blood cell analyzer of any one of claims 1 to 7, **characterized in that**, calculating at least one leukocyte parameter of at least one leukocyte particle population in the test sample from the optical information and obtaining an infection marker parameter based on the at least one leukocyte parameter by the processor comprises:

obtain a plurality of parameters of at least one leukocyte particle population in the test sample from the optical information,
obtain a plurality of sets of infection marker parameters for determining whether the subject has a severe infection from the plurality of parameters, and
calculate a credibility of each set of infection marker parameters of the plurality of sets of infection marker parameters, select at least one set of infection marker parameters from the plurality of sets of infection marker parameters based on respective credibility of the plurality of sets of infection marker parameters so as to obtain the infection marker parameter.

**16.** The blood cell analyzer of any one of claims 1 to 7, **characterized in that**, calculating at least one leukocyte parameter of at least one leukocyte particle population in the test sample from the optical information and obtaining an infection marker parameter based on the at least one leukocyte parameter by the processor comprises:

determine whether the blood sample to be tested has an abnormality that affects the determination of the likelihood of progression to sepsis within the certain period of time according to the optical information; and
when it is determined that the blood sample to be tested has an abnormality that affects the determination of the likelihood of progression to sepsis within the certain period of time, obtain at least one leukocyte parameter of at least one leukocyte particle population unaffected by the abnormality from the optical information so as to obtain the infection marker parameter.

**17.** The blood cell analyzer of any one of claims 1 to 16, **characterized in that**, the diagnostic efficacy of the infection marker parameter is greater than 0.5, preferably greater than 0.6, and more preferably greater than 0.8.

**18.** A blood cell analyzer, **characterized in that** the blood cell analyzer comprises:

a measurement device configured to mix a blood sample of a subject to be tested, a hemolytic agent and a staining agent to prepare a test sample and perform optical measurement on the test sample to obtain optical information of the test sample; and
a controller configured to:

receive a mode setting instruction,
when the mode setting instruction indicates that a blood routine test mode is selected, control the measurement device to perform optical measurement on a first measurement amount of a test sample at a first measurement amount to obtain optical information of the test sample, and obtain and output blood routine parameters of the test sample based on said optical information,
when the mode setting instruction indicates that a sepsis test mode is selected, control the measurement device to perform optical measurement on a second measurement amount of a test sample, the second measurement amount being greater than the first measurement amount, to obtain optical information of the test sample, calculate at least one leukocyte parameter of at least one leukocyte particle population in the test sample from said optical information, obtain an infection marker parameter based on the at least one leukocyte parameter, and output the infection marker parameter, wherein the infection marker parameter is used for predicting whether the subject is likely to progress to sepsis within a certain period of time starting from when the blood sample to be tested is collected.

**19.** A method for predicting whether a subject is likely to progress to sepsis in the future, **characterized in that** the method comprises:

obtaining a blood sample to be tested from the subject;
preparing a test sample containing a part of the blood sample to be tested, a hemolytic agent, and a staining agent for leukocyte classification;
passing particles in the test sample through an optical detection region irradiated with light one by one, so as to obtain optical information generated by the particles in the test sample after being irradiated with light;
calculating at least one leukocyte parameter of at least one leukocyte particle population in the test sample from the optical information;
obtaining an infection marker parameter based on the at least one leukocyte parameter; and
predicting whether the subject is likely to progress to sepsis in a certain period of time in the future based on the infection marker parameter.

**20.** An infection marker parameter for use in predicting whether a subject is likely to progress to sepsis in the future, **characterized in that** the infection marker parameter is obtained by:

obtaining at least one leukocyte parameter of at least one leukocyte particle population obtained by flow cytometry detection of a test sample containing a part of a blood sample to be tested from the subject, a hemolytic agent, and a staining agent for leukocyte classification; and
obtaining the infection marker parameter based on the at least one leukocyte parameter.

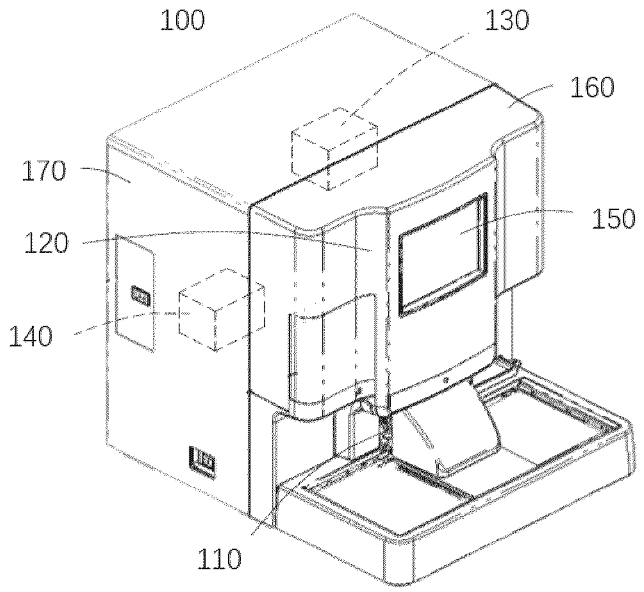


FIG. 1

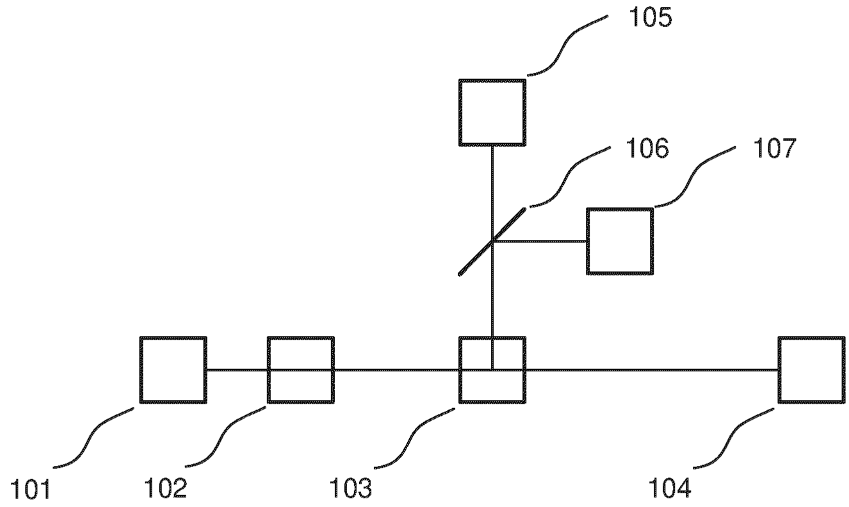


FIG. 2

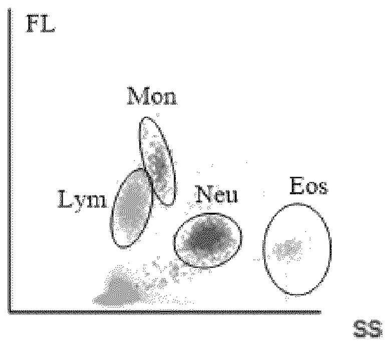


FIG. 3

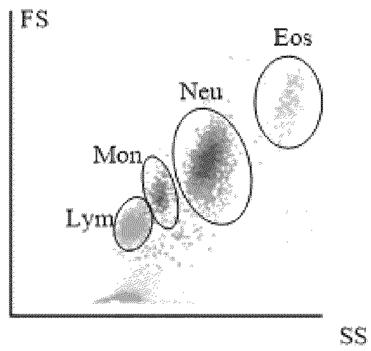


FIG. 4

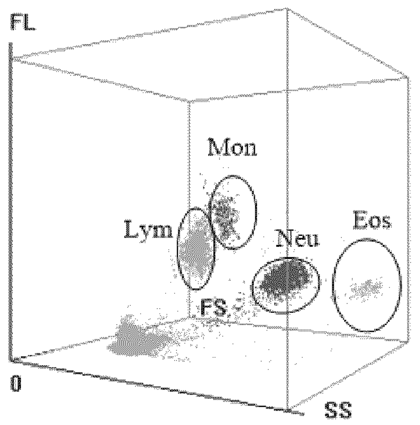


FIG. 5

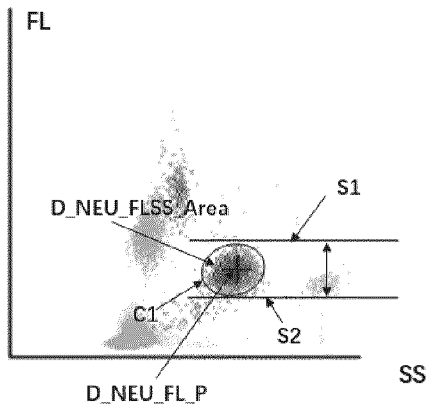


FIG. 6

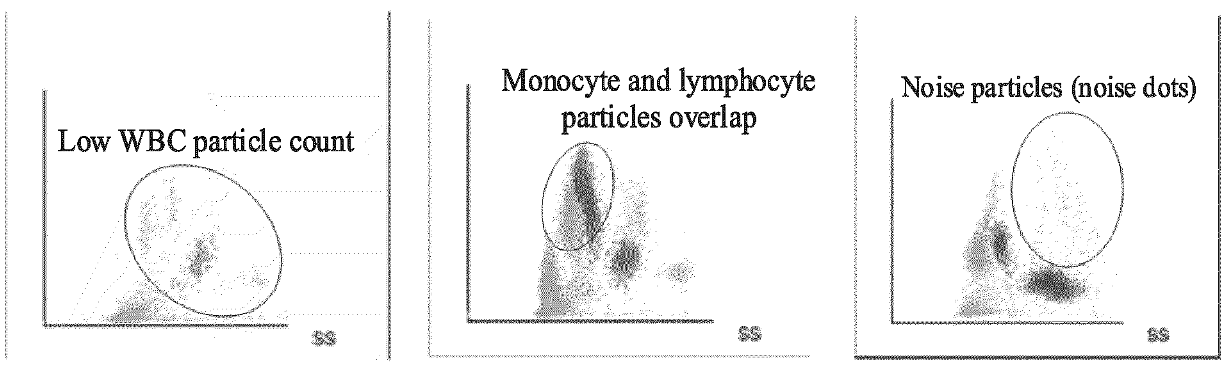


FIG. 7(a) FIG. 7(b) FIG. 7(c)

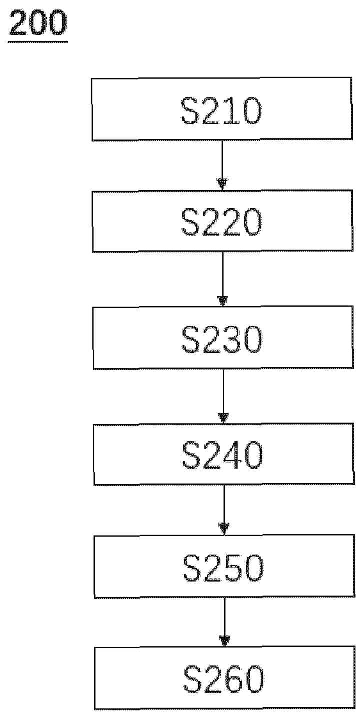
200
S210
S220
S230
S240
S250
S260

*FIG. 8*

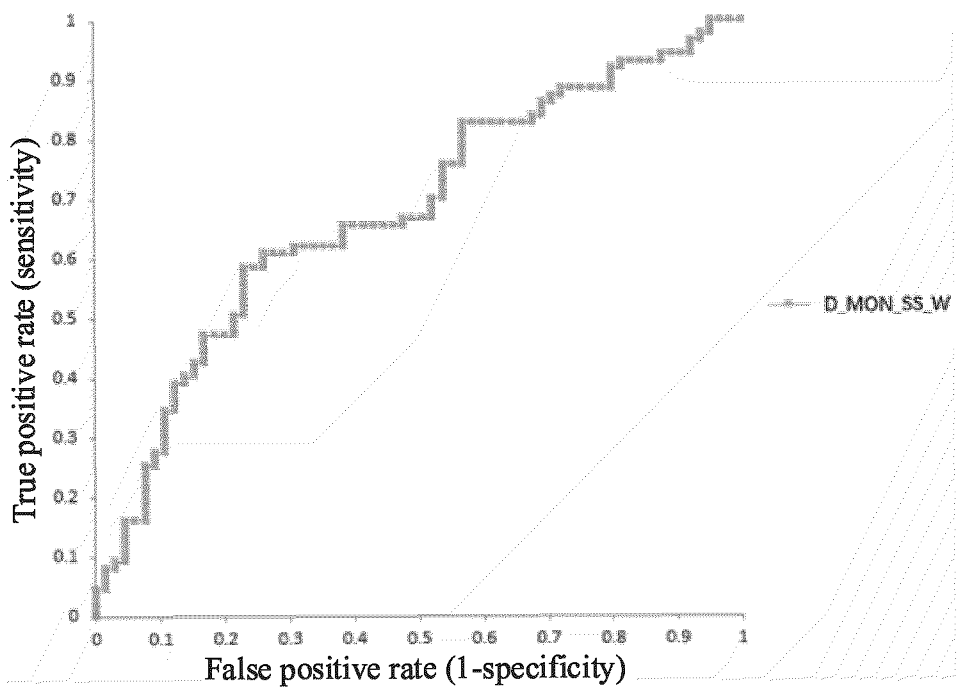
True positive rate (sensitivity)
False positive rate (1-specificity)
D_MON_SS_W
0
0.1
0.2
0.3
0.4
0.5
0.6
0.7
0.8
0.9
1

*FIG. 9*

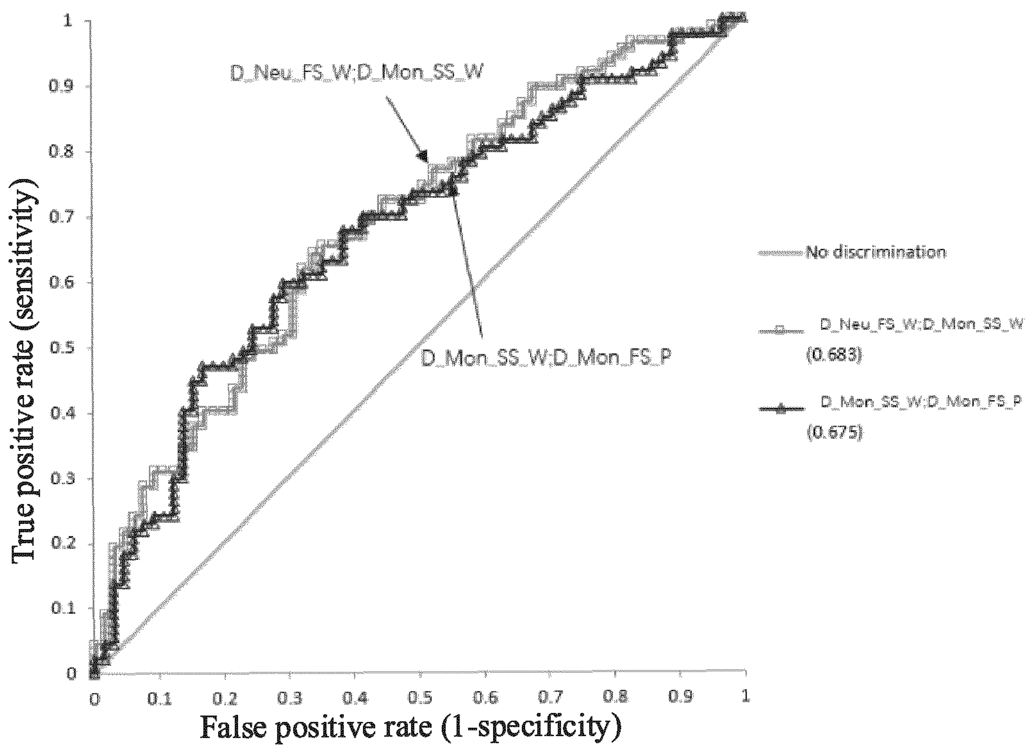
D_Neu_FS_W;D_Mon_SS_W
D_Mon_SS_W;D_Mon_FS_P
No discrimination
D_Neu_FS_W;D_Mon_SS_W
(0.683)
D_Mon_SS_W;D_Mon_FS_P
(0.675)
True positive rate (sensitivity)
False positive rate (1-specificity)
0
0.1
0.2
0.3
0.4
0.5
0.6
0.7
0.8
0.9
1

*FIG. 10*

Step-by-step schematic diagram:

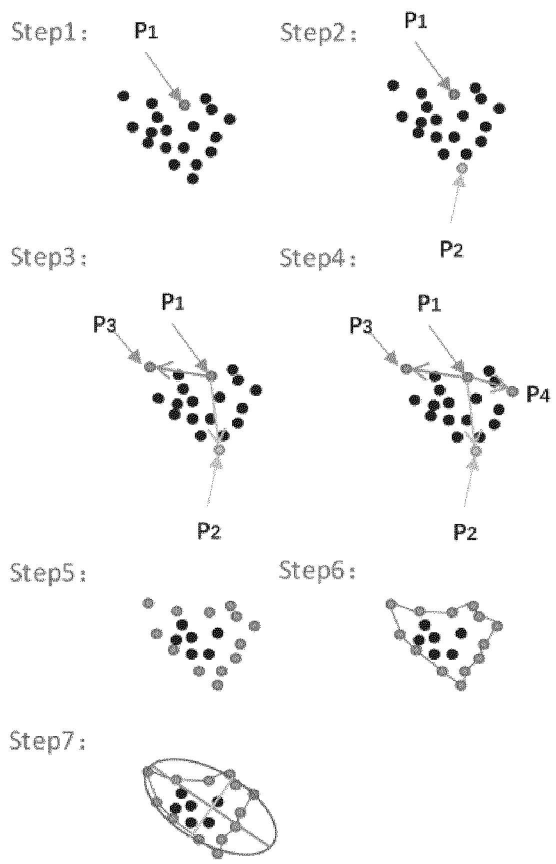


*FIG. 11*

| INTERNATIONAL SEARCH REPORT | International application No. **PCT/CN2022/143971** |
|---|---|

**A. CLASSIFICATION OF SUBJECT MATTER**

G01N 33/49(2006.01)i;G01N 15/00(2006.01)i;G01N 21/64(2006.01)i

According to International Patent Classification (IPC) or to both national classification and IPC

**B. FIELDS SEARCHED**

Minimum documentation searched (classification system followed by classification symbols)

IPC：G01N

Documentation searched other than minimum documentation to the extent that such documents are included in the fields searched

Electronic data base consulted during the international search (name of data base and, where practicable, search terms used)

CNPAT, CNKI, EPODOC, WPI: 深圳迈瑞生物医疗电子股份有限公司, 李进, 张晓梅, 潘世耀, 马玉帅, 祁欢, 叶燚, 血液, 细胞, 白细胞, 脓毒症, 中性粒细胞, 单核细胞, blood, cell, leukocyte, sepsis, neutrophil, monocyte

**C. DOCUMENTS CONSIDERED TO BE RELEVANT**

| Category* | Citation of document, with indication, where appropriate, of the relevant passages | Relevant to claim No. |
|---|---|---|
| Y | CN 111602052 B (SHENZHEN MINDRAY BIO-MEDICAL ELECTRONICS CO., LTD.) 12 October 2021 (2021-10-12)<br>description, paragraphs [0008]-[0480], and figures 1-24 | 1-18 |
| Y | CN 104777109 A (AFFILIATED CHILDREN'S HOSPITAL OF CAPITAL INSTITUTE OF PEDIATRICS et al.) 15 July 2015 (2015-07-15)<br>description, paragraphs [0004]-[0043] | 1-18 |
| A | CN 109030432 A (SYSMEX CORPORATION) 18 December 2018 (2018-12-18)<br>entire document | 1-18 |
| A | CN 111418023 A (BECKMAN COULTER, INC.) 14 July 2020 (2020-07-14)<br>entire document | 1-18 |
| A | CN 111542744 A (SHENZHEN MINDRAY BIO-MEDICAL ELECTRONICS CO., LTD.) 14 August 2020 (2020-08-14)<br>entire document | 1-18 |
| A | WO 2021216752 A1 (BECKMAN COULTER INC.) 28 October 2021 (2021-10-28)<br>entire document | 1-18 |

☑ Further documents are listed in the continuation of Box C. ☑ See patent family annex.

* Special categories of cited documents:

"A" document defining the general state of the art which is not considered to be of particular relevance

"D" document cited by the applicant in the international application

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **10 March 2023** | **20 March 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/143971**

**C. DOCUMENTS CONSIDERED TO BE RELEVANT**

| Category* | Citation of document, with indication, where appropriate, of the relevant passages | Relevant to claim No. |
|---|---|---|
| A | WO 9945094 A1 (COMPUCYTE CORP.) 10 September 1999 (1999-09-10)<br>entire document | 1-18 |

Form PCT/ISA/210 (second sheet) (July 2022)

**INTERNATIONAL SEARCH REPORT**

International application No.
**PCT/CN2022/143971**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19-20**
because they relate to subject matter not required to be searched by this Authority, namely:

Claims 19-20 relate to the diagnosis of sepsis, wherein whether a subject is likely to develop into sepsis is predicted according to infection marker parameters. Therefore, claims 19-20 belong to a disease diagnosis method.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/143971**

| Patent document cited in search report | | | Publication date (day/month/year) | Patent family member(s) | | | Publication date (day/month/year) |
|---|---|---|---|---|---|---|---|
| CN | 111602052 | B | 12 October 2021 | EP | 3789764 | A1 | 10 March 2021 |
| | | | | WO | 2019206300 | A1 | 31 October 2019 |
| | | | | US | 2021102935 | A1 | 08 April 2021 |
| CN | 104777109 | A | 15 July 2015 | None | | | |
| CN | 109030432 | A | 18 December 2018 | US | 2018356328 | A1 | 13 December 2018 |
| | | | | US | 10895522 | B2 | 19 January 2021 |
| | | | | JP | 2018205274 | A | 27 December 2018 |
| | | | | JP | 6903494 | B2 | 14 July 2021 |
| | | | | EP | 3413046 | A1 | 12 December 2018 |
| | | | | EP | 3413046 | B1 | 09 September 2020 |
| CN | 111418023 | A | 14 July 2020 | EP | 3701541 | A1 | 02 September 2020 |
| | | | | WO | 2019084361 | A1 | 02 May 2019 |
| | | | | US | 2019128906 | A1 | 02 May 2019 |
| CN | 111542744 | A | 14 August 2020 | None | | | |
| WO | 2021216752 | A1 | 28 October 2021 | None | | | |
| WO | 9945094 | A1 | 10 September 1999 | AU | 2884499 | A | 20 September 1999 |
| | | | | EP | 1066368 | A1 | 10 January 2001 |
| | | | | JP | 2002505441 | A | 19 February 2002 |
| | | | | CA | 2321203 | A1 | 10 September 1999 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019206300 A1
- CN 101750274 A